(19) Europäisches Patentamt
European Patent Office
Office européen des brevets

(11) **EP 4 678 639 A1**

(12) **EUROPEAN PATENT APPLICATION**
published in accordance with Art. 153(4) EPC

(43) Date of publication:
**14.01.2026 Bulletin 2026/03**

(21) Application number: **24785379.9**

(22) Date of filing: **08.04.2024**

(51) International Patent Classification (IPC):
***C07D 405/12*** *(2006.01)* ***A61K 31/4184*** *(2006.01)*
***A61K 9/20*** *(2006.01)* ***A61K 9/00*** *(2006.01)*

(52) Cooperative Patent Classification (CPC):
**A61K 9/00; A61K 9/20; A61K 31/4184; C07D 405/12**

(86) International application number:
**PCT/KR2024/004667**

(87) International publication number:
**WO 2024/210689 (10.10.2024 Gazette 2024/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.04.2023 KR 20230046284**
**03.11.2023 KR 20230151217**

(71) Applicant: **JW Pharmaceutical Corporation**
**Gyeonggi-do 13840 (KR)**

(72) Inventors:
- **JUNG, Jae Hoon**
  **Gwangju-si, Gyeonggi-do 12777 (KR)**
- **YOON, Doo Ha**
  **Seongnam-si, Gyeonggi-do 13562 (KR)**
- **PARK, Kyung Yee**
  **Seoul 07015 (KR)**
- **CHIN, Sei Ho**
  **Hwaseong-si, Gyeonggi-do 18297 (KR)**
- **UM, Yun Sik**
  **Gwangmyeong-si, Gyeonggi-do 14306 (KR)**
- **KIM, Yang Hee**
  **Hwaseong-si, Gyeonggi-do 18374 (KR)**
- **CHOI, Su Yeon**
  **Seoul 03074 (KR)**

(74) Representative: **Croce, Valeria**
**Jacobacci & Partners S.p.A.**
**Via Senato, 8**
**20121 Milano (IT)**

# (54) SALT OF BENZIMIDAZOLE DERIVATIVE

(57) Provided are a hydrochloride or hydrochloride hydrate of a compound of Formula 1, and a pharmaceutical composition including the same.

FIG. 2A

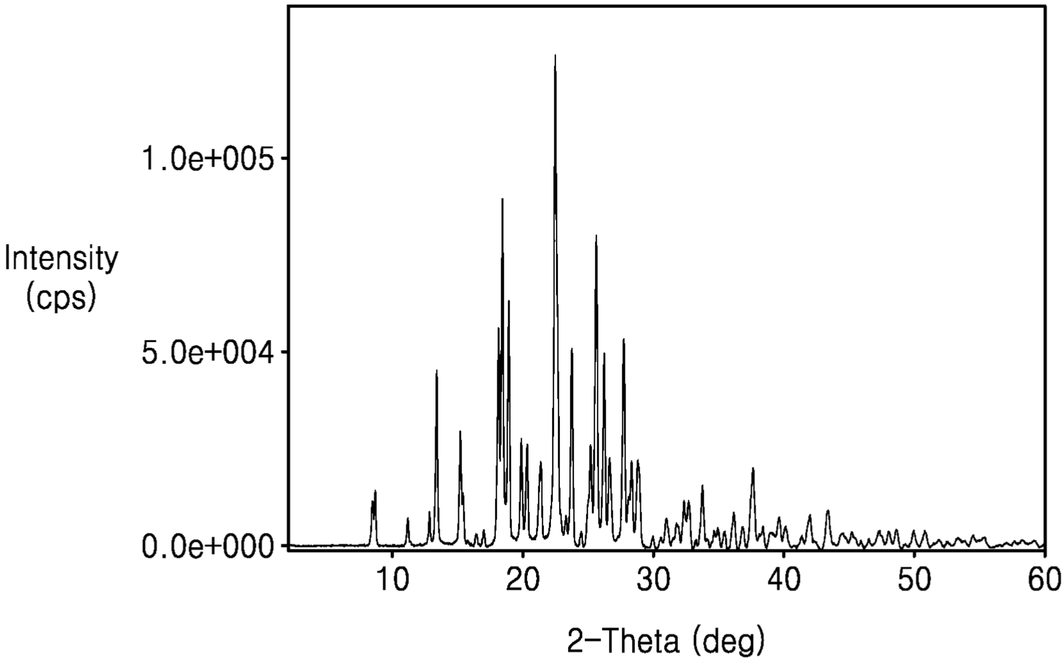
1.0e+005
5.0e+004
0.0e+000
Intensity
(cps)
10
20
30
40
50
60
2-Theta (deg)

## Description

### Technical Field

The present disclosure relates to a novel acid addition salt of a benzimidazole derivative, and more specifically, to a novel acid addition salt of a benzimidazole derivative having high stability, sufficient aqueous solubility, and high bioavailability regardless of food intake.

### Background Art

Acid pump inhibitors (H+/K+ ATPase inhibitors) are known to be effective in the treatment of gastrointestinal diseases by inhibiting acid secretion in the gastrointestinal tract through reversible potassium-competitive inhibition of H+/K+ ATPase. The acid pump inhibitors also referred to as potassium-competitive acid blockers (PCABs) to distinguish them from proton pump inhibitors. Chromane-substituted benzimidazole derivative compounds have been studied as substances effective as acid pump inhibitors (WO2007/072146). Among them, 4-[((4S)-5,7-difluoro-3,4-dihydro-2H-chromen-4-yl)oxy]-N,N,2-trimethyl-1H-benzo[d]imidazole-6-carboxamide] represented by the following Formula 1 is a pharmaceutically active ingredient with a molecular weight of 387.39, and is also well known by the generic name tegoprazan.

[Formula 1]

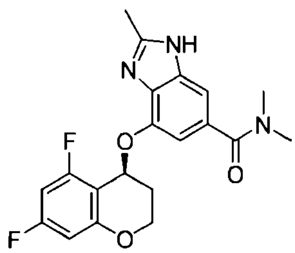

The compound has preventive and therapeutic effects on diseases mediated by acid pump inhibitory activity. Gastrointestinal diseases mediated by such acid pump inhibitory activity include, but are not limited to, gastroesophageal disease, gastroesophageal reflux disease (GERD), peptic ulcer, gastric ulcer and duodenal ulcer, ulcer induced by NSAIDs, gastritis, Helicobacter pylori infection, dyspepsia, functional dyspepsia, Zollinger-Ellison syndrome, non-erosive reflux disease (NERD), visceral pain, heartburn, nausea, esophagitis, dysphagia, hypersalivation, airway disorder and asthma (WO2007/072146).

The crystalline form A of the freebase of the compound of Formula 1 is disclosed in International Patent Publication No. WO2016/117814. According to this, it is disclosed that crystalline form A has excellent light stability and low hygroscopicity and static electricity generation, making it advantageous for formulation. Crystalline form A of the freebase of the compound of Formula 1 is currently being marketed in the form of oral tablets (product name: K-CAB tablets) and orally disintegrating tablets (product name: K-CAB orally disintegrating tablets) with approval from the Ministry of Food and Drug Safety of the Republic of Korea.

International Patent Publication WO2018/056697 discloses pidolate and malate salts of the compound of Formula 1, and discloses that they have superior aqueous solubility and stability compared to the freebase amorphous form of the compound of Formula 1, and therefore may be advantageously used as a raw material for oral formulations and injectable formulations.

The number of salts applicable to a single compound is very large and polymorphs may exist, and depending on the type and polymorph of the salt, the solubility, stability, etc. may vary when formulated as a pharmaceutical, and these physicochemical properties are important factors that determine the quality of the pharmaceutical. Therefore, even if a previously known compound or a specific salt or crystalline form thereof is known, much research and effort is required to develop salts and crystalline forms having improved physicochemical properties.

[Prior Art Documents]

[Patent Documents]

(Patent Document 001) WO2007/072146
(Patent Document 002) WO2016/117814
(Patent Document 003) WO2018/056697

## Disclosure of Invention

### Technical Problem

One aspect is to provide an acid addition salt of a compound of Formula 1 having excellent pharmaceutical properties such as aqueous solubility, stability, and the like.

Another aspect is to provide a crystalline form of an acid addition salt of the compound of Formula 1 having excellent pharmaceutical properties such as aqueous solubility, stability, and the like.

Another aspect is to provide a pharmaceutical composition including an acid addition salt or a crystalline form of an acid addition salt of the compound of the Formula 1 as an active ingredient.

Other objects and advantages of the present application will become more apparent from the following detailed description taken together with the appended claims. Any matter not described in the present application is sufficiently recognizable and inferable to a person of ordinary skill in the art of the present application or a similar technical field, and therefore, its description is omitted.

### Solution to Problem

One aspect provides a salt, which is a hydrochloride salt or hydrochloride hydrate of a compound of the following Formula 1:

[Formula 1]

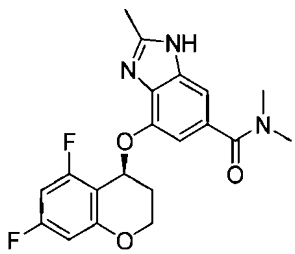

Another aspect provides a pharmaceutical composition including a hydrochloride salt or hydrochloride hydrate of a compound of Formula 1 and a pharmaceutically acceptable carrier.

Another aspect provides a hydrochloride salt or hydrochloride hydrate of the compound of Formula 1 for use in the prevention or treatment of diseases mediated by acid pump inhibitory activity.

### Advantageous Effects of Invention

According to an aspect of the present disclosure, the hydrochloride salt or hydrochloride hydrate of a compound of Formula 1 has been confirmed to have a higher solubility at least 10 times or more compared to that of the existing commercially available freebase crystalline form A, and thus is expected to secure sufficient bioavailability when administered to a living body, and in fact, it has been confirmed that a formulation formulated in the same manner as the existing commercial formulation has an equivalent or higher bioavailability. In addition, the hydrochloride salt or hydrochloride hydrate has significantly better overall liquid stability, solid stability, and photostability compared to other types of acid addition salts including known acid addition salts, and in particular, the hydrochloride hydrate showed the best stability in all aspects, and showed significantly better stability compared to other acid addition salts even when combined with pharmaceutically acceptable additives.

Furthermore, when the hydrochloride hydrate orally disintegrating tablet, which is prepared in the same manner as the commercial orally disintegrating tablet, was evaluated for its disintegration time against the commercial formulation (K-CAB orally disintegrating tablet), and it showed remarkably faster disintegration characteristics and is therefore expected to provide superior patient convenience in oral administration and a faster onset of action. In addition, the hydrochloride hydrate tablets, which were prepared in the same manner as the commercial formulation, were compared in a dissolution test with the commercial tablets (K-CAB tablets), both showed similar high immediate-release characteristics of 85 % or more within 15 minutes under pH 1.2 conditions corresponding to the fasting state, however, under pH 4.0, pH

6.8, and FaSSiF solution conditions, the dissolution of the reference drug was significantly delayed, but the hydrochloride hydrate formulation (test drug) still showed a high rate of 85 % or more within 15 minutes. Therefore, when a tablet is prepared using hydrochloride hydrate, it is expected that immediate-release properties may be maintained regardless of changes in gastrointestinal pH and delays in gastrointestinal emptying time due to meals, thereby ensuring stable high bioavailability regardless of the time of administration, and thus improving ease of administration.

**Brief Description of Drawings**

FIG. 1 illustrates powder X-ray diffraction (PXRD) results and differential scanning calorimetry (DSC) results of the previously known freebase crystalline form A of the compound of Formula 1 (WO2016/117814).
FIG. 2 illustrates PXRD and DSC results of the crystalline form A of the hydrochloride hydrate of the compound of Formula 1.
FIG. 3 illustrates PXRD and DSC results of a hydrochloride salt (amorphous form) of the compound of Formula 1.
FIG. 4 illustrates PXRD and DSC results of a hydrobromide salt of the compound of Formula 1.
FIG. 5 illustrates PXRD and DSC results of a malate salt of the compound of Formula 1.
FIG. 6 illustrates PXRD and DSC results of a pidolate salt of the compound of Formula 1.
FIG. 7 illustrates PXRD results (FIG. 7A), DSC results (FIG. 7B), and PXRD results (FIG. 7C) of a sulfate salt of the compound of Formula 1 before (top) and after (bottom) one month of storage under stress stability conditions (RH 90 %).
FIG. 8 illustrates PXRD and DSC results of a phosphate salt of the compound of Formula 1.
FIG. 9 illustrates PXRD and DSC results of a maleate salt of the compound of Formula 1.
FIG. 10 illustrates PXRD and DSC results of a methanesulfonate salt (amorphous form) of the compound of Formula 1.
FIG. 11 illustrates PXRD and DSC results of a methanesulfonate salt (crystalline form) of the compound of Formula 1.
FIG. 12 illustrates PXRD and DSC results of a hemisalicylate salt of the compound of Formula 1.
FIG. 13 is a graph illustrating the results of measuring the total amount of related substances after performing a stress stability test (60 °C or 90 % RH) on tegoprazan acid addition salts.
FIG. 14 illustrates the results of measuring the total related substances after performing a compatibility test on a hydrate crystalline form of tegoprazan hydrochloride with various pharmaceutical additives (at 60 °C or 90 % RH for 1 week or 3 weeks).
FIG. 15 illustrates the results of measuring the total related substances after performing a compatibility test of a tegoprazan sulfate salt with various pharmaceutical additives (at 60 °C or 90 % RH for 1 week or 3 weeks).
FIG. 16 illustrates the results of measuring the total related substances after performing a compatibility test of a crystalline form of a tegoprazan methanesulfonate salt with various pharmaceutical additive (at 60 °C or 90 % RH for 1 week or 3 weeks).
FIG. 17 is a graph illustrating the results of measuring the weight change after preparing purified and orally disintegrating tablets for each of tegoprazan acid addition salts and storing them under stress storage conditions (90 % RH) for 3 weeks.
FIG. 18 is a graph illustrating the results of measuring the total related substances after preparing a tablet for each of tegoprazan acid addition salts and performing a stress stability test (60 °C or 90 % RH).
FIG. 19 is a graph illustrating the results of measuring the total related substances after preparing an orally disintegrating tablet for each of tegoprazan acid addition salts and performing a stress stability test (60 °C or 90 % RH).
FIG. 20 illustrates photographs taken at the time point when the wetting time was measured, after preparing the orally disintegrating tablet for each of the tegoprazan acid addition salts, placing it in 18 mL of a saline solution at about 37 °C and pH 6.8, and allowing the entire surface of the orally disintegrating tablet to be wetted by the solution.
FIG. 21 is a graph illustrating the results of a comparative dissolution test performed on tegoprazan hydrochloride hydrate (crystalline form) tablets in a pH 1.2 solution and a pH 4.0 solution, using 50 mg K-CAB tablets as a reference drug.
FIG. 22 is a graph illustrating the results of a comparative dissolution test performed on tegoprazan hydrochloride hydrate (crystalline form) tablets in a pH 6.8 solution and FaSSiF solution, using 50 mg K-CAB tablets as a reference drug.
FIG. 23 is a graph illustrating the results of a pharmacokinetic test conducted on beagle dogs, using 50 mg K-CAB tablets as a reference drug for tegoprazan hydrochloride hydrate (crystalline form) tablets.

**Mode for the Invention**

Hereinafter, the present disclosure is described in more detail.

All technical terms used in the present disclosure, unless otherwise defined, are used as commonly understood by one of ordinary skill in the relevant field of the present disclosure. In addition, while suitable methods or samples are described herein, similar or equivalent methods are also included within the scope of the present disclosure.

One aspect provides a salt, which is a hydrochloride salt or hydrochloride hydrate of a compound of the following Formula 1:

[Formula 1]

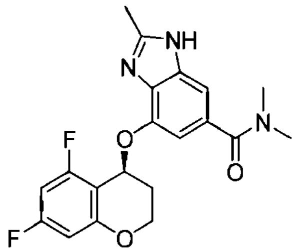

In the present application, the compound of the Formula 1 is also referred to as '4-[((4S)-5,7-difluoro-3,4-dihydro-2H-chromen-4-yl)oxy]-N,N,2-trimethyl-1H-benzimidazole-6-carboxamide' or by the generic name 'tegoprazan.'

The freebase of the compound of the Formula 1 may be prepared according to the method described in WO2007/072146 or WO2016/117814 (Patent Document 2), which are incorporated herein by reference in their entirety. The salt according to the aspect may be prepared by preparing the freebase of the compound of Formula 1 and then using any method for preparing the hydrochloride salt or hydrochloride hydrate from the freebase, and in an embodiment, may be prepared according to the method disclosed in Example 1 (hydrochloride hydrate) or Example 2 (hydrochloride salt).

The term "freebase" is used herein for convenience to refer to the parent compound represented by Formula 1, to distinguish it from any salt.

The hydrochloride salt of the compound of the Formula 1 has the following Formula 2:

[Formula 2]

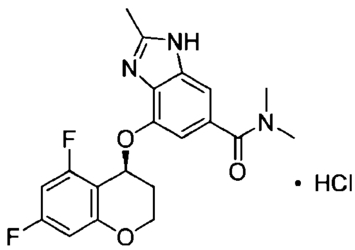

The hydrochloride salt of the Formula 2 may be amorphous.

In an embodiment, the amorphous form may be observed to have one endothermic peak at about 189.91 °C when measured by DSC (scan rate 10 °C/min) (FIG. 3B).

In an embodiment, the hydrochloride salt amorphous form shows an endothermic peak with an onset of about 179.64 °C when measured by DSC (scan rate 10 °C/min) (FIG. 3B).

In an embodiment, the hydrochloride salt amorphous form may be characterized by including an endothermic peak having an onset at about 179.64 °C and a minimum at about 189.91 °C as measured by DSC (scan rate 10 °C/min) (FIG. 3B).

The hydrochloride salt amorphous form was confirmed to have excellent stability as it produced significantly less related substances compared to other salts in an accelerated stability test (40 $\pm$ 2 °C/RH 75 $\pm$ 5 %) and a stress stability test condition (60 °C or 90 % RH). Therefore, the hydrochloride salt amorphous form is suitable for pharmaceutical use and is expected to ensure the stability of pharmaceuticals when prepared into pharmaceuticals.

The hydrate of the tegoprazan hydrochloride salt has the following Formula 3:

[Formula 3]

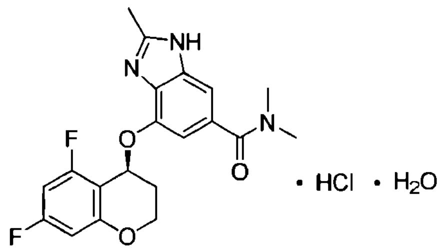

The hydrochloride hydrate of the Formula 3 may be in crystalline form. In an embodiment, the hydrochloride hydrate of the Formula 3 is in crystalline form A.

In an embodiment, the hydrochloride hydrate crystalline form A may have a powder X-ray diffraction (PXRD) pattern including peaks at diffraction angles 20 of 18.4±0.2°, 22.5±0.2°, and 25.6±0.2°.

In an embodiment, the hydrochloride hydrate crystalline form A may have a powder X-ray diffraction (PXRD) pattern including peaks at diffraction angles 20 of 13.4±0.2°, 15.2±0.2°, 18.4±0.2°, 18.9±0.2°, 22.5±0.2°, 23.7±0.2°, 25.6±0.2°, 26.2±0.2°, and 27.7±0.2°.

In an embodiment, the hydrochloride hydrate crystalline form A may have a powder X-ray diffraction (PXRD) pattern including peaks at diffraction angles 20 of 8.7±0.2°, 13.4±0.2°, 15.2±0.2°, 18.1±0.2°, 18.4±0.2°, 18.9±0.2°, 19.9±0.2°, 20.3±0.2°, 21.4±0.2°, 22.5±0.2°, 23.7±0.2°, 25.2±0.2°, 25.6±0.2°, 26.2±0.2°, 26.6±0.2°, 27.7±0.2° 28.3 ±0.2°, 28.8±0.2°, and 37.5±0.2°.

The peak at the diffraction angle 20 is a powder X-ray diffraction (PXRD) pattern when irradiated with X-rays (Cu(ka), 1.54056 Å).

In an embodiment, the hydrochloride hydrate crystalline Form A may have a powder X-ray diffraction (PXRD) pattern substantially consistent with FIG. 2A when irradiated with X-rays (Cu(ka)).

In an embodiment, the hydrochloride hydrate crystalline form A may have a powder X-ray diffraction (PXRD) pattern as shown in FIG. 2A when irradiated with X-rays (Cu(ka)).

In an embodiment, the hydrochloride hydrate crystalline Form A may have a powder X-ray diffraction (PXRD) pattern substantially consistent with Table 1 below when irradiated with X-rays (Cu(ka)).

[Table 1]

| **2θ(±0.2)** | **d** | **$I/I_o$(%)** | **2θ(±0.2)** | **d** | **$I/I_o$ (%)** |
|---|---|---|---|---|---|
| 8.7 | 10.18 | 6.34 | 23.7 | 3.74 | 30.43 |
| 13.4 | 6.61 | 25.75 | 25.2 | 3.53 | 11.21 |
| 15.2 | 5.82 | 16.72 | 25.6 | 3.47 | 56.47 |
| 18.1 | 4.89 | 33.88 | 26.2 | 3.40 | 31.16 |
| 18.4 | 4.81 | 48.38 | 26.6 | 3.35 | 18.21 |
| 18.9 | 4.68 | 34.79 | 27.7 | 3.22 | 34.30 |
| 19.9 | 4.46 | 16.83 | 28.3 | 3.15 | 8.43 |
| 20.3 | 4.37 | 15.96 | 28.8 | 3.10 | 22.26 |
| 21.4 | 4.15 | 17.49 | 37.5 | 2.40 | 12.24 |
| 22.5 | 3.95 | 100.00 | | | |
| 2θ: diffraction angle, d: distance between crystal planes, $I/I_0$: relative intensity (where "I" represents the intensity of each peak; "$I_o$" represents the intensity of the highest peak.) | | | | | |

In an embodiment, the hydrochloride hydrate crystalline form A may be observed to have an endothermic peak at about 181.94 °C when measured by DSC (scan rate 10 °C/min) (FIG. 2B).

In an embodiment, the hydrochloride hydrate crystalline form A shows a sharp endothermic peak with an onset of about 176.5 °C when measured by DSC (scan rate 10 °C/min) (FIG. 2B).

In an embodiment, the hydrochloride hydrate crystalline form A may be characterized by including an endothermic peak having an onset at about 176.5 °C and a minimum at about 181.94 °C when measured by DSC (scan rate 10 °C min) (FIG. 2B).

The analytical instrument and measurement method for the PXRD analysis according to an embodiment are as follows.

(1) Powder X-ray Diffraction (PXRD)

Powder X-ray diffraction (PXRD) analysis was performed by continuous θ-2θ scanning over a range of about 2° to 60° in 2θ at a scan rate of 5°/min (step size 0.02° 20).

(2) Differential Scanning Calorimetry (DSC)

Differential scanning calorimetry (DSC) was performed from 25 °C to 400 °C. A sample of 1 to 2 mg was weighed into an aluminum DSC pan, and the sample was heated from 25 °C to 400 °C at a scan rate of 10 °C/min to monitor the heat flow response (DSC) generated.

In an embodiment, the hydrochloride hydrate crystalline form A may be stably stored without transformation into another crystalline form under general pharmaceutical storage conditions.

In an embodiment, the hydrochloride hydrate crystalline form A allows for stable storage of the pharmaceutical under accelerated stability test (40 ± 2 °C/RH 75 ± 5 %) and stress stability test conditions (60 °C or 90 % RH).

The compound of the Formula 1, in other words, tegoprazan, is known to generate related substances including a degradant of the following Formula 4 during storage or formulation, especially in an acidic environment where solubility increases (WO2018/056697). Therefore, the smaller the amount of the degradant of Formula 4 and the amount of total related substances including it, the more pharmaceutically stable it may be considered.

[Formula 4]

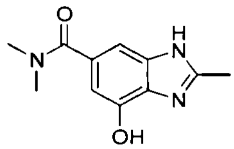

In the present application, the compound of the Formula 4 is also referred to as a 'degradant of Formula 4,' or simply as a 'degradant.'

As a result of the test, it was confirmed that the tegoprazan hydrochloride hydrate crystalline form and the tegoprazan hydrochloride salt amorphous form according to an embodiment have a higher solubility at least 10 times or more compared to that of the tegoprazan freebase crystalline form commercially available as K-CAB tablets, and thus it is expected that sufficient absorption will occur when administered to a living body (see Experimental Example 1). In addition, as a result of the liquid stability test, the hydrochloride hydrate crystalline form and the hydrochloride salt amorphous form were confirmed to have remarkably superior stability compared to other acid addition salts, with an increase in the degradant of Formula 4 of only 0.5 % or less (see Experimental Example 2), and as a result of the solid stability test, the hydrochloride hydrate crystalline form was confirmed to have remarkably superior stability compared to other acid addition salts, with a very low increase in the total related substances and degradant of Formula 4 throughout the accelerated stability test and the stress stability test (see Experimental Example 3). In addition, the hydrochloride hydrate crystalline form showed significantly better stability compared to other acid addition salts in photostability tests (see Experimental Example 4). In addition, it was confirmed that the hydrochloride salt amorphous form had overall superior liquid stability, solid stability, and photostability compared to other acid addition salts (see Experimental Examples 2, 3, and 4).

Although methanesulfonate salt (amorphous form, crystalline form) was very stable in a liquid stability test, with an increase in degradants of 0.5 % or less, it was confirmed to have low stability in solid stability tests, as the total related substances and degradants significantly increased, or the substance absorbed moisture and deliquesced. Although the previously disclosed malate salt and pidolate salt had high solubility, in the liquid stability test, solids were precipitated after 24 hours, and in the solid stability test under stress conditions of 90 % RH, they were unstable as they melted within 24 hours of the start of the test, and in the photostability test, compared to tegoprazan hydrochloride hydrate, a large amount of total related substances and degradants were generated, making them unstable. In contrast, the hydrochloride salt amorphous form and hydrochloride hydrate crystalline form did not show phenomena such as solid precipitation or melting under the same conditions, and the degradants were also generated in a significantly low content, making them stable (see Experimental Examples 2, 3, and 4). Therefore, the stability test results showed that tegoprazan hydrochloride salt amorphous form and hydrochloride hydrate crystalline form showed significantly superior stability overall compared to other acid addition salts. In particular, it was confirmed that tegoprazan hydrochloride hydrate crystalline form showed

significantly better stability compared to other acid addition salts, and also had significantly better stability compared to the previously disclosed malate salt and pidolate salt. In addition, based on a comprehensive comparison of solubility, liquid stability, solid-state stability, and photostability, it may be said that the hydrochloride hydrate crystalline form and the hydrochloride salt amorphous form are pharmaceutically excellent acid addition salts that are easy to store as raw pharmaceutical materials and have stability even after being prepared into pharmaceuticals.

Another aspect of the present disclosure provides a hydrochloride salt or hydrochloride hydrate of the compound of Formula 1 for use in the prevention or treatment of diseases mediated by acid pump inhibitory activity.

Another aspect of the present disclosure provides a pharmaceutical composition including a hydrochloride salt or hydrochloride hydrate of the compound of the Formula 1, and a pharmaceutically acceptable additive. The hydrochloride salt or hydrochloride hydrate of the Formula 1 may be specifically a hydrochloride salt amorphous form of Formula 2 or a hydrochloride hydrate crystalline form of Formula 3. The hydrochloride salt or hydrochloride hydrate of the Formula 1 is as described above for the hydrochloride salt or hydrochloride hydrate of the compound of Formula 1 according to the aspect.

The pharmaceutical composition may be used for the prevention or treatment of diseases mediated by acid pump inhibitory activity.

The diseases mediated by the acid pump inhibitory activity may be selected from the group consisting of gastroesophageal disease, gastroesophageal reflux disease (GERD), peptic ulcer, gastric ulcer, duodenal ulcer, ulcer induced by NSAID, gastritis, Helicobacter pylori infection, dyspepsia, functional dyspepsia, Zollinger-Ellison syndrome, non-erosive reflux disease (NERD), visceral referred pain, heartburn, nausea, esophagitis, dysphagia, hyper-salivation, airway disorder, and asthma.

The pharmaceutical composition may be formulated into a formulation selected from the group consisting of powder, granule, tablet, orally disintegrating tablet, capsule, suspension, emulsion, syrup, aerosol, ointment, cream, suppository, and injectable formulation.

In an embodiment, the pharmaceutical composition is an oral tablet or an orally disintegrating tablet.

In an embodiment, the pharmaceutical composition is a tablet or orally disintegrating tablet containing a crystalline form of a hydrochloride hydrate represented by Formula 3 as an active ingredient.

In an embodiment, the pharmaceutical composition is an orally disintegrating tablet containing a crystalline form of a hydrochloride hydrate represented by Formula 3 as an active ingredient.

As a result of the study, the hydrochloride hydrate crystalline form was formulated in the same manner as the commercial tegoprazan freebase orally disintegrating tablet (K-CAB orally disintegrating tablet) and the disintegration time was evaluated compared to the commercial formulation (K-CAB orally disintegrating tablet), and it showed remarkably fast disintegration characteristics, and is therefore, expected to provide superior patient convenience in oral administration and a faster onset of action (see Experimental Example 7-4).

In an embodiment, the pharmaceutical composition is an oral tablet containing a crystalline form of a hydrochloride hydrate represented by Formula 3 as an active ingredient.

The hydrochloride hydrate crystalline form was formulated in the same manner as the commercial tablet (K-CAB tablet) containing tegoprazan freebase crystalline form A, and as a result of a comparative dissolution test with the commercial tablet (K-CAB tablet), both showed similarly high immediate-release characteristics of 85 % or more within 15 minutes under pH 1.2 conditions corresponding to the fasting state, and under pH 4.0, pH 6.8, and FaSSiF solution conditions, the reference drug (K-CAB tablet) showed significantly delayed dissolution, whereas the hydrochloride hydrate crystalline form formulation still showed high release characteristics of 85 % or more within 15 minutes (see Experimental Examples 7-5 and 7-6). Even in conditions where the pH in the stomach fluctuates (increases) after a meal, delaying dissolution, or where the drug release is delayed due to this, and the formulation reaches the intestinal (duodenal or small intestine) environment beyond the gastric fluid environment, the hydrochloride hydrate crystalline form formulation shows immediate-release, allowing it to show rapid effects and high bioavailability. Therefore, when a tablet is prepared using a hydrochloride hydrate crystalline form, immediate-release properties may be maintained regardless of changes in gastrointestinal pH due to meals, and thus a stable high bioavailability may be secured, and therefore, a rapid effect and high bioavailability may be achieved not only when taken on an empty stomach before a meal but also after a meal (in other words, regardless of whether it is administered after a meal). Accordingly, it is expected that ease of administration may be enhanced.

Furthermore, as a result of an in vivo -study conducted on animals, it was confirmed that the tablet of the hydrochloride hydrate crystalline form shortens the $T_{max}$ with improved solubility while securing a similar bioavailability (systemic exposure level) compared to the reference drug, thereby enabling rapid absorption of the drug, which may lead to a more rapid onset of action (see Experimental Example 7-7).

In an embodiment, the pharmaceutically acceptable additive may vary depending on the intended formulation, and it is well known in the art which additives may be specifically used depending on the formulation.

In an embodiment, the pharmaceutical composition is an oral tablet, and specifically, may further include one or more substances selected from the group consisting of a diluent, a binder, a disintegrant, and a lubricant as the pharmaceutically acceptable additive. Any additive known to be commonly used in the art may be used as the diluent,

binder, disintegrant, and lubricant, but the stability and dissolution rate of the active ingredient may be further improved by selecting a specific additive. As the additive, the diluent may be selected from the group consisting of lactose, starch, mannitol, microcrystalline cellulose, carboxymethylcellulose, and any combination thereof, but is not limited thereto; the binder may be selected from the group consisting of povidone, hypromellose, hydroxypropyl cellulose, copovidone, and any combination thereof, but is not limited thereto; the disintegrant may be selected from the group consisting of crospovidone, croscarmellose sodium, sodium starch glycolate, low-substituted hydroxypropylcellulose, and any combination thereof, but is not limited thereto; and the lubricant may be selected from the group consisting of magnesium stearate, talc, light anhydrous silicic acid, sodium stearyl fumarate, and any combination thereof, but is not limited thereto.

In an embodiment, the oral tablet may include, but is not limited to, hydroxypropyl cellulose, mannitol, microcrystalline cellulose, croscarmellose sodium, light anhydrous silicic acid, and magnesium stearate.

In an embodiment, the pharmaceutical composition is an orally disintegrating tablet and includes a diluent, a binder, a disintegrant, a lubricant, and the like. In an embodiment, the oral tablet may include, but is not limited to, mannitol, sucralose, pearlitol flash, crospovidone, maltitol, enzyme-treated stevia, peppermint, light anhydrous silicic acid, and magnesium stearate.

The amount of active ingredient per unit formulation contained in the pharmaceutical composition varies depending on the condition of the patient to be administered, the degree of treatment intended, etc. Preferably, the composition of the present disclosure may include the freebase of the compound represented by Formula 1 as an active ingredient in an amount of 1 to 100 mg, preferably in an amount of 1 to 50 mg, for example, in an amount of about 50 mg.

The selection of additives and specific formulation methods for the pharmaceutical compositions may be appropriately performed by a person of ordinary skill in the art using knowledge known in the art, and reference may be made to, for example, Remington's Pharmaceutical Science (latest edition), Mack Publishing Company, Easton PA.

Hereinafter, the composition and effects of the present disclosure will be described in more detail through examples. However, the following examples are only illustrative of the present disclosure and the content of the present disclosure is not limited by the following examples.

**Test Method**

**Powder X-ray Diffraction (PXRD) Test Method**

PXRD measurements were performed using an X-ray diffractometer (Rigaku MiniFlex 600). The measurements were taken under the following conditions.

**[Analysis Conditions]**

| | |
|---|---|
| X-ray generator | Target: Cu(Ka) |
| Detector | D/teX Ultra |
| Sample holder | Glass holder |
| Tube voltage | 40 kV |
| Tube current | 15 mA |
| Slit condition | Variable+Fixed slit system |
| Optical devices | |
| Soller(inc.) | 5.0 deg |
| HIS | 10.0 mm |
| DS | 1.250 deg |
| SS | 13.0 mm(open) |
| Soller(rec.) | 5.0 deg |
| RS | 13.0 mm(open) |
| Monochromatization | None |

(continued)

| Measurement condition | |
|---|---|
| Scan axis | Theta/2-Theta |
| Mode | Continuous |
| Start (deg) | 2.0 |
| Stop(deg) | 60.0 |
| Step(deg) | 0.02 |
| Speed(deg/min) | 5.0 |
| Spin | On |

Measurement Method: a sample was added to a glass holder and measured according to the analysis conditions.

DSC Test Method

Differential Scanning Calorimeter (DSC) was measured using a temperature differential scanning calorimeter (SCINCO DSC N-650). The measurements were taken under the following conditions.

**[Analysis Conditions]**

| | | | | |
|---|---|---|---|---|
| Sample fan | Aluminum(closed) | | | |
| Sample amount | 1 ~ 2 mg | | | |
| Gas | $N_2$ | | | |
| Sample rate | 1.00 | | | |
| Temp. Program | Start Temp. | End Temp. | Heating rate | Hold time |
| | 25 °C | 400 °C | 10.0 °C/min | 0 min |

Measurement method: a sample of 1 to 2 mg was weighed and placed in an aluminum sample pan, which was then sealed with a lid to prepare the sample. An aluminum DSC pan without a sample was sealed with a lid and used as a reference. The sample and the reference were placed in the furnace, the lids were closed, and the measurement was initiated once the analysis start temperature was stably maintained.

Related Substance Test Method (HPLC)

The amount of degradants of Formula 4 and total related substances was measured using liquid chromatography (HPLC), and in the present disclosure, Infinity 1260 (Agilent) equipment was used. The analysis conditions are as follows.

**[Solution Preparation]**

Blank Solution (Diluent)

400 mL of water and 600 mL of acetonitrile were mixed.

Standard Solution

Accurately weighed 50.0 mg of tegoprazan freebase was placed in a 50 mL volumetric flask, dissolved with the diluent, and then made up to the mark with the diluent. 1 mL of this solution was taken and diluted to 25 mL to prepare a standard solution (0.04 mg/mL).

Test Solution

A test sample (100.0 mg as tegoprazan freebase) was accurately weighed and placed in a 25 mL volumetric flask, dissolved, and then made up to the mark with the diluent to prepare a test solution (4.0 mg/mL).

**[Device Conditions]**

| | | | |
|---|---|---|---|
| Detector | | Ultraviolet absorbance spectrophotometer (measurement wavelength: 261 nm) | |
| Column | Capcellpak MG C18 (4.6 mm X 150 mm, 3 μm) or a similar column | | |
| Column Temperature | 40 °C | | |
| Mobile Phase A | pH 4.3 buffer solution:<br><br>0.63 g of ammonium formate ($NH_4HCO_2$) was dissolved in 1000 mL of water, and the pH was adjusted to 4.3 with formic acid. | | |
| Mobile Phase B | B: pH 4.3 buffer: acetonitrile (5:95):<br><br>0.63 g of ammonium formate ($NH_4HCO_2$) was dissolved in 50 mL of water, and the pH was adjusted to 4.3 with formic acid. This solution was mixed with 950 mL of acetonitrile. | | |
| Gradient Condition | Time (Minutes) | Mobile Phase A (%) | Mobile Phase B (%) |
| | 0.0 | 100 | 0 |
| | 2.0 | 100 | 0 |
| | 10.0 | 65 | 35 |
| | 16.0 | 65 | 35 |
| | 34.0 | 20 | 80 |
| | 42.0 | 20 | 80 |
| | 42.1 | 100 | 0 |
| | 60.0 | 100 | 0 |
| Flow rate | 1.0 mL/min | | |
| Injection Volume | 2.5 μL | | |
| Analysis Time | 60 Minutes | | |

Operation and Calculation: The blank solution, standard solution, and test solution were sequentially tested under the instrument operating conditions according to the liquid chromatography method in the General Test Methods of the 12th Edition of the Korean Pharmacopoeia, and the amount of related substances was calculated using the following equation.

$$\text{Amount of each related substance (\%)} = \frac{A_T}{A_S} \times \frac{C_S}{C_T} \times \frac{\text{Molecular weight of the sample}}{\text{Molecular weight of the standard product}} \times P \times \frac{1}{RRF}$$

$A_T$ = Area of each related substance peak obtained from the test solution
$A_S$ = Area of the tegoprazan peak obtained from the standard solution
$C_S$ = Concentration of standard solution (mg/mL)
$C_T$ = Concentration of test solution (mg/mL)
P = Purity/Assay/Potency (%) of standard product
RRF = Relative response factor (1.0)

The amount of total related substances were calculated as the sum of the amounts of individual related substances.

**Example**

Tegoprazan acid addition salts were prepared using various acids shown in Table 2 below. In addition, tegoprazan malate salt and tegoprazan pidolate salt were prepared according to the method disclosed in Korean Patent No. 1829706.

[Table 2]

| Acid | Tegoprazan Acid Addition Salt | Crystalline Form | Chemical Structures of Acid Addition Salts |
|---|---|---|---|
| Hydrochloric Acid | Hydrochloride Hydrate | Crystalline Form | 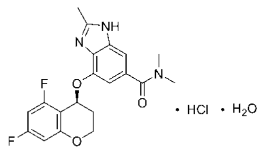 |
| | Hydrochloride Salt | Amorphous Form | 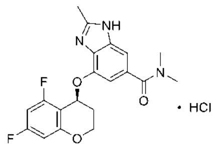 |
| Hydrobromic Acid | Hydrobromide Salt | Crystalline Form | 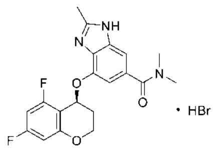 |
| Sulfuric Acid | Sulfate Salt | Crystalline Form | 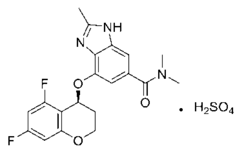 |
| Phosphoric Acid | Phosphate Salt | Amorphous Form | 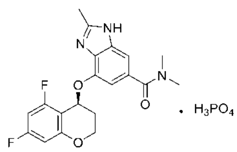 |
| Maleic Acid | Maleate Salt | Amorphous Form | 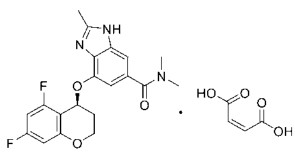 |

(continued)

| Acid | Tegoprazan Acid Addition Salt | Crystalline Form | Chemical Structures of Acid Addition Salts |
|---|---|---|---|
| Methanesulfonic Acid | Methanesulfonate Salt | Amorphous Form | 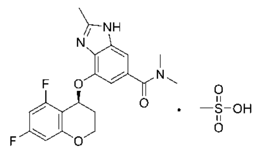 |
| | Methanesulfonate Salt | Crystalline Form | 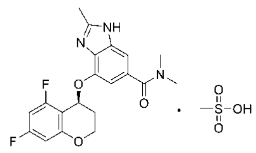 |
| Salicylic Acid | Hemisalicylate Salt | Amorphous Form | 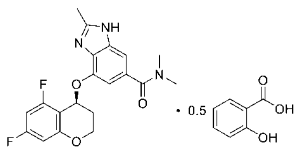 |
| L-Malic Acid | Malate Salt | Amorphous Form | 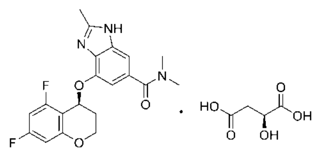 |
| L-Pyroglutamic Acid | Pidolate Salt | Amorphous Form | 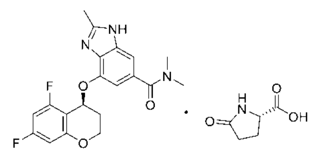 |

**Preparation Example 1. Preparation of Tegoprazan freebase Crystalline Form A**

1.5 kg of 7-hydroxy-N,N,2-trimethyl-3-(p-tolylsulfonyl)benzimidazole-5-carboxamide, 822.5 g (1.1 equiv.) of (4R)-5,7-difluorochromen-4-ol, and 15 L of tetrahydrofuran were added into a reactor, and a solution of 1.04 kg (1.2 equiv.) of diisopropyl azodicarboxylate diluted in 3 L of tetrahydrofuran was slowly added dropwise. After completion of charging, the mixture was stirred for 2 hours and then concentrated under reduced pressure at an external temperature of 40 °C. After cooling the concentrated solution to room temperature, 7.5 L of diethyl ether was added and stirred at room temperature for 18 hours. The solid was filtered, washed with 5 L of diethyl ether, and the wet mass (3.26 kg), 6.52 L of isopropyl alcohol, and 9.78 L of diethyl ether were added into the reactor and stirred at room temperature for 3 hours. The solid was filtered, washed with 5 L of diethyl ether, and vacuum dried at 40 °C to obtain 7-[(4S)-5,7-difluorochromen-4-yl]oxy-N,N,2-trimethyl-3-(p-tolylsulfonyl)benzimidazole-5-carboxamide (1.43 kg, yield 64.86 %).

1.43 kg of 7-[(4*S*)-5,7-difluorochromen-4-yl]oxy-*N*,*N*,2-trimethyl-3-(*p*-tolylsulfonyl)benzimidazole-5-carboxamide, 3.85 L of tetrahydrofuran, 12.97 L of isopropyl alcohol, and 12.97 L of 2N aqueous sodium hydroxide solution were added into the reactor and stirred at room temperature for 4 hours. 14.3 L of ethyl acetate was added to the reaction solution, stirred for 10 minutes, allowed to stand for 10 minutes, and then the layers were separated. To the obtained organic layer, 14.3 L of 10 % aqueous ammonium chloride solution was added, stirred for 10 minutes, allowed to stand for 10 minutes, and then the layers were separated. To the obtained organic layer, 142.5 g of magnesium sulfate was added, stirred for 5 minutes, and then filtered. The volume of ethyl acetate was concentrated at an external temperature of 50 °C until about 3 times the amount of starting substance remained. After stirring at room temperature for 18 hours, the solid was filtered, washed with 7.13 L of ethyl acetate, and vacuum dried at 40 °C to obtain crude tegoprazan (746.56 g, yield 73.24 %).

746.56 g of crude tegoprazan was dissolved in 2.24 L of methanol and filtered. The mixture was stirred and concentrated at an external temperature of 50 °C, 2.24 L of ethyl acetate was added, and the mixture was slowly cooled to room temperature while stirring for 12 hours. The solid was filtered, washed with 1.49 L of ethyl acetate, and vacuum dried at 40 °C to obtain tegoprazan freebase crystalline form A (665.26 g, yield: 89.11 %). The PXRD and DSC results of the prepared tegoprazan hydrochloride hydrate are shown in FIG. 1.

$^1$H NMR (500 MHz, DMSO-$d_6$) δ ppm 2.01 - 2.13 (m, 1 H) 2.25 (br d, *J*=15.41 Hz, 1 H) 2.41 - 2.40 (m, 3 H) 2.98 (br d, *J*=11.44 Hz, 6 H) 4.20 - 4.31 (m, 1 H) 4.37 (br s, 1 H) 5.80 (br s, 1 H) 6.18 (br s, 1 H) 6.71 (br t, *J*=10.83 Hz, 1 H) 6.79 - 6.00 (m, 2 H) 7.05 (s, 1 H) 7.10 (s, 1 H) 7.18 (s, 1 H) 12.41 (s, 1 H)

Acid addition salts in the following examples and comparative examples were prepared using the prepared tegoprazan freebase crystalline form A (hereinafter referred to as 'tegoprazan') as a starting substance.

**Example 1. Preparation of Hydrochloride Hydrate of Compound of Formula** 1

20 g of tegoprazan and 660 mL of acetonitrile were added into the reactor and cooled to 0 to 5 °C. 4.878 mL (1.1 equiv.) of hydrochloric acid was slowly added dropwise. After the solid was completely dissolved and solid precipitation began, the mixture was stirred at 0 to 5 °C for 2 hours. The solid was filtered, washed with 100 mL of acetonitrile, and vacuum dried at room temperature to obtain tegoprazan hydrochloride hydrate as a white powder (20.16 g, yield 88.21 %). The PXRD and DSC results of the prepared tegoprazan hydrochloride hydrate are shown in FIG. 2.

$^1$H NMR (500 MHz, DMSO-$d_6$) δ ppm 2.10 - 2.18 (m, 1 H) 2.21 - 2.20 (m, 1 H) 2.74 (s, 3 H) 2.88 - 3.12 (m, 6 H) 4.27 - 4.37 (m, 1 H) 4.37 - 4.44 (m, 1 H) 6.08 (br s, 1 H) 6.72 - 6.77 (m, 1 H) 6.87 (td, *J*=0.61, 2.44 Hz, 1 H) 7.37 (d, *J*=0.76 Hz, 1 H) 7.44 (s, 1 H)

**Example 2. Preparation of Hydrochloride Salt (Amorphous Form) of Compound of Formula 1**

20 g of tegoprazan was dissolved in 660 mL of methanol and cooled to 0 to 5 °C. 4.434 mL (1.0 equiv.) of hydrochloric acid was slowly added at 0 to 5 °C and was concentrated at an external temperature of 50 °C until a solid precipitated. 200 mL of acetone and 400 mL of diethyl ether were added and stirred vigorously at room temperature for 1 hour. The solid was filtered, washed with 100 mL of a mixture of acetone: diethyl ether (1:2, v/v), and vacuum dried at 40 °C to obtain tegoprazan hydrochloride salt (amorphous form) as a white powder (20.32 g, yield 92.86 %). The PXRD and DSC results of the prepared tegoprazan hydrochloride salt amorphous form are shown in FIG. 3.

$^1$H NMR (500 MHz, DMSO-$d_6$) δ ppm 2.00 - 2.18 (m, 1 H) 2.21 - 2.20 (m, 1 H) 2.72 (s, 3 H) 3.01 (d. *J*=1.00 Hz. 6 H) 4.25 - 4.35 (m. 1 H) 4.40 (br d, *J*=10.99 Hz, 1 H) 6.07 (br s. 1 H) 6.74 (br d, *J*=10.38 Hz, 1 H) 6.87 (td, *J*=9.57, 2.37 Hz, 1 H) 7.36 (d, *J*=0.61 Hz, 1 H) 7.41 (s, 1 H)

**Comparative Example 1. Preparation of Hydrobromide Salt of Compound of Formula 1**

30 g of tegoprazan and 900 mL of acetonitrile were added into the reactor and cooled to 0 to 5 °C. 20.07 mL (1.1 equiv.) of hydrobromic acid was slowly added dropwise. After the solid was completely dissolved and solid precipitation began, the mixture was stirred at 0 to 5 °C for 17 hours. The solid was filtered, washed with 150 mL of acetonitrile, and dried at 40 °C to obtain tegoprazan hydrobromide salt as a white powder (24.66 g, yield 67.87 %). The PXRD and DSC results of the prepared tegoprazan hydrobromide salt are shown in FIG. 4.

$^1$H NMR (500 MHz, DMSO-$d_6$) δ ppm 2.12 - 2.21 (m, 1 H) 2.23 - 2.28 (m, 1 H) 2.73 (s. 3 H) 3.00 (d. *J*=1.00 Hz, 6 H) 4.20 - 4.20 (m. 1 H) 4.42 (br d, *J*=11.14 Hz, 1 H) 6.09 (br s. 1 H) 6.76 (br d, *J*=10.38 Hz, 1 H) 6.89 (td, *J*=0.61, 2.44 Hz, 1 H) 7.41 (d, *J*=0.92 Hz, 1 H) 7.47 (s, 1 H)

**Comparative Example 2. Preparation of Malate Salt of Compound of Formula 1**

15 g of tegoprazan and 5.45 g (1.05 equiv.) of L-malic acid were completely dissolved in 600 mL of methanol and concentrated at an external temperature of 50 °C until a solid precipitated. 600 mL of diethyl ether was added and stirred vigorously at room temperature for 30 minutes. The solid was filtered, washed with 75 mL of diethyl ether, and vacuum dried at 40 °C to obtain tegoprazan malate salt as a white powder (18.79 g, yield 93.06 %). The PXRD and DSC results of the prepared tegoprazan malate salt are shown in FIG. 5.

$^1$H NMR (500 MHz, DMSO-$d_6$) δ ppm 2.02 - 2.14 (m, 1 H) 2.25 (dd, *J*=15.18, 2.06 Hz. 1 H) 2.41 - 2.40 (m, 4 H) 2.62 (dd. *J*=15.72. 4.88 Hz, 1 H) 2.98 (br s. 6 H) 4.22 - 4.20 (m. 2 H) 4.37 (br d. *J*=10.99 Hz, 1 H) 6.04 (br s, 1 H) 6.71 (br d, *J*=10.38 Hz, 1 H) 6.84 (td, *J*=9.57, 2.52 Hz, 1 H) 6.94 (s, 1 H) 7.14 (s, 1 H)

**Comparative Example 3. Preparation of Pidolate Salt of Compound of Formula 1**

15 g of tegoprazan and 5.25 g (1.05 equiv.) of L-pyroglutamic acid were completely dissolved in 600 mL of methanol and concentrated at an external temperature of 50 °C until a solid precipitated. 600 mL of n-hexane was added and stirred vigorously at room temperature for 30 minutes. The solid was filtered, washed with 75 mL of *n*-hexane, and vacuum dried at 40 °C to obtain tegoprazan pidolate salt as a white powder (19.18 g, yield 95.91 %). The PXRD and DSC results of the prepared tegoprazan pidolate salt are shown in FIG. 6.

$^1$H NMR (500 MHz, DMSO-$d_6$) δ ppm 1.93 - 2.01 (m, 1 H) 2.04 - 2.10 (m, 3 H) 2.22 - 2.37 (m, 2 H) 2.46 (s. 3 H) 2.98 (br s, 6 H) 4.06 (ddd, *J*=9.00, 4.27, 0.61 Hz, 1 H) 4.20 - 4.30 (m, 1 H) 4.37 (br d. *J*=10.99 Hz, 1 H) 6.04 (br s, 1 H) 6.71 (br d. *J*=10.38 Hz, 1 H) 6.84 (td, *J*=0.54, 1.98 Hz. 1 H) 6.93 (br s, 1 H) 7.14 (br s, 1 H) 7.92 (s, 1 H) 12.10 - 13.17 (m, 2 H)

**Comparative Example 4. Preparation of Sulfate Salt of Compound of Formula 1**

20 g of tegoprazan and 400 mL (1 equiv.) of acetone were added into the reactor and cooled to 15 °C. 2.767 mL (1.0 equiv.) of sulfuric acid was slowly added dropwise at 15 °C and stirred at 15 °C for 19 hours. The solid was filtered, washed with 100 mL of acetone, and vacuum dried at 40 °C to obtain tegoprazan sulfate salt as an apricot-colored powder (24.25 g, yield 96.58 %). The PXRD and DSC results of the prepared tegoprazan sulfate salt, and the polymorphic transition results at the initial time point and after 1 month of the stress stability (90 % RH) test are shown in FIG. 7.
$^1$H NMR (500 MHz, DMSO-$d_6$) δ ppm 2.11 - 2.22 (m, 1 H) 2.23 - 2.20 (m, 1 H) 2.72 (s, 3 H) 2.00 - 3.13 (m, 5 H) 4.21 - 4.28 (m, 1 H) 4.42 (br d, *J*=11.14 Hz, 1 H) 6.08 (br s, 1 H) 6.76 (br d, *J*=10.38 Hz, 1 H) 6.89 (td, *J*=9.61, 2.44 Hz, 1 H) 7.30 - 7.43 (m, 1 H) 7.45 (s. 1 H)

**Comparative Example 5. Preparation of Phosphate Salt of Compound of Formula 1**

20 g of tegoprazan and 1,000 mL of isopropyl alcohol were added into the reactor. After the solid was completely dissolved, 2.65 mL (0.84 equiv.) of phosphoric acid was slowly added dropwise. After concentrating until a solid precipitated at an external temperature of 50 °C, 200 mL of acetone and 400 mL of diethyl ether were added and stirred vigorously at room temperature for 1 hour. The solid was filtered, washed with 100 mL of a mixture of acetone: diethyl ether (1:2, v/v), and vacuum dried at 40 °C to obtain tegoprazan phosphate salt as a white powder (19.62 g, yield 85.16 %). The PXRD and DSC results of the prepared tegoprazan phosphate salt are shown in FIG. 8.
$^1$H NMR (500 MHz, DMSO-$d_6$) δ ppm 2.11 - 2.22 (m, 1 H) 2.23 - 2.20 (m, 1 H) 2.72 (s, 3 H) 2.90 - 3.13 (m, 5 H) 4.21 - 4.28 (m, 1 H) 4.42 (br d, *J*=11.14 Hz, 1 H) 6.08 (br s, 1 H) 6.76 (br d, *J*=10.38 Hz, 1 H) 6.89 (td, *J*=9.61, 2.44 Hz, 1 H) 7.39 - 7.43 (m, 1 H) 7.45 (s. 1 H)

**Comparative Example 6. Preparation of Maleate Salt of Compound of Formula 1**

20 g of tegoprazan and 800 mL of dichloromethane were added into the reactor and cooled to 0 to 5 °C. After the solid was completely dissolved, 5.99 g (1.0 equiv.) of maleic acid was added and the temperature was raised to room temperature to completely dissolve it. After concentrating until a solid precipitated at room temperature, 800 mL of diethyl ether was added and stirred vigorously at room temperature for 2 hours. The solid was filtered, washed with 100 mL of diethyl ether, and vacuum dried at 60 °C to obtain tegoprazan maleate salt as a white powder (24.87 g, yield 95.50 %). The PXRD and DSC results of the prepared tegoprazan maleate salt are shown in FIG. 9.
$^1$H NMR (500 MHz, DMSO-$d_6$) δ ppm 2.07 - 2.16 (m, 1 H) 2.25 (dd. *J*=15.18, 1.91 Hz, 1 H) 2.57 (s, 3 H) 2.99 (br s. 6 H) 4.19 - 4.27 (m, 1 H) 4.30 (br d, *J*=10.99 Hz, 1 H) 6.05 (br s, 1 H) 6.17 (s, 2 H) 6.72 (br d, *J*=10.38 Hz, 1 H) 6.85 (td, *J*=9.61, 2.44 Hz, 1 H) 7.17 (s, 1 H) 7.25 - 7.27 (m, 1 H)

**Comparative Example 7. Preparation of Methanesulfonate Salt (Amorphous Form) of Compound of Formula 1**

20 g of tegoprazan was completely dissolved in 200 mL of methanol, and 3.35 mL (1.0 equiv.) of methanesulfonic acid was added dropwise. The mixture was concentrated until a solid precipitated at an external temperature of 40 °C, 800 mL of diethyl ether was added, and the mixture was stirred vigorously at room temperature for 1 hour. The solid was filtered, washed with 100 mL of diethyl ether, and vacuum dried at 40 °C to obtain tegoprazan methanesulfonate salt (amorphous form) as a white powder (24.45 g, yield 97.95 %). The PXRD and DSC results of the prepared tegoprazan methanesulfonate salt (amorphous form) are shown in FIG. 10.
$^1$H NMR (500 MHz, DMSO-$d_6$) δ ppm 2.11 - 2.21 (m, 1 H) 2.23 - 2.30 (m, 1 H) 2.35 (s, 3 H) 2.73 (s, 3 H) 3.00 (d, *J*=1.00 Hz, 6 H) 4.20 - 4.32 (m, 1 H) 4.42 (br d, *J*=11.14 Hz, 1 H) 6.08 (br s, 1 H) 6.76 (br d, *J*=10.38 Hz, 1 H) 6.88 (td, *J*=9.61, 2.44 Hz, 1 H) 7.42 (s, 1 H) 7.46 (s, 1 H)

**Comparative Example 8. Preparation of Methanesulfonate Salt (Crystalline Form) of Compound of Formula 1**

50 g of tegoprazan and 1,000 mL of acetonitrile were placed in a reactor, and 8.4 mL (1.0 equiv.) of methanesulfonic acid was added dropwise at room temperature. After the solid was completely dissolved and began to precipitate, it was stirred at room temperature for 1 hour. The solid was filtered, washed with 250 mL of acetonitrile, and vacuum dried at 40 °C to obtain tegoprazan methanesulfonate salt (crystalline form) as a white powder (59.41 g, yield 95.20 %). The PXRD and DSC results of the prepared tegoprazan methanesulfonate salt (crystalline form) are shown in

FIG. 11.
$^1H$ NMR (500 MHz, DMSO-$d_6$) δ ppm 2.12 - 2.22 (m, 1 H) 2.23 - 2.28 (m, 1 H) 2.33 (s, 3 H) 2.72 (s, 3 H) 2.89 - 3.10 (m, 6 H) 4.21 - 4.20 (m, 1 H) 4.42 (br d, *J*=11.14 Hz, 1 H) 6.08 (br s, 1 H) 6.76 (br d, *J*=10.38 Hz, 1 H) 6.80 (td, *J*=9.54, 2.44 Hz, 1 H) 7.40 - 7.42 (m, 1 H) 7.45 (s, 1 H)

**Comparative Example 9. Preparation of Hemisalicylate Salt of Compound of Formula 1**

20 g of tegoprazan was completely dissolved in 200 mL of methanol, and 4.272 g (0.6 equiv.) of salicylic acid was added. After the solid was completely dissolved, the mixture was concentrated at an external temperature of 40 °C until the solid precipitated, 400 mL of diethyl ether was added, and the mixture was stirred vigorously for 1 hour. The solid was filtered, washed with 100 mL of diethyl ether, and vacuum dried at 40 °C to obtain tegoprazan hemisalicylate salt as a white powder (18.82 g, yield 79.86 %). The PXRD and DSC results of the prepared tegoprazan hemisalicylate salt are shown in FIG. 12.
$^1H$ NMR (500 MHz, DMSO-$d_6$) δ ppm 2.04 - 2.13 (m, 1 H) 2.25 (dd, *J*=15.11, 1.98 Hz, 1 H) 2.48 (s, 3 H) 2.98 (br s, 6 H) 4.21 - 4.20 (m, 1 H) 4.37 (br d, *J*=10.99 Hz, 1 H) 6.04 (br s, 1 H) 6.71 (br d, *J*=10.38 Hz, 1 H) 6.84 (td, *J*=9.61, 2.44 Hz, 1 H) 6.87 - 6.94 (m, 1 H) 6.98 (s, 1 H) 7.15 - 7.18 (m, 1 H) 7.47 (ddd, *J*=8.43, 7.13, 1.68 Hz, 1 H) 7.78 (dd, *J*=7.78, 1.68 Hz, 1 H)

**Experimental Example**

The following experiments were conducted on the above-mentioned prepared tegoprazan acid addition salt.
Solubility tests and stability tests were conducted, and the conditions were as shown in Table 3 below.

[Table 3]

| Tests | Test Condition | Packaging Condition | Test Period | Analytic al Method |
|---|---|---|---|---|
| Accelerated Stability | 40 ± 2 °C / RH 75 ± 5 % | Double PE bag and Al bag | 1 Month | HPLC, XRD |
| Stress Stability | Temperature (60 °C) | Amber Vial (closed) | | |
| | Humidity (90 % RH) | Clear Vial (opened) | | |
| Photostability | Ultraviolet Light (UV): 200 W·hr/m$^2$ Visible Light: 1.2 Mlux·hr | PE-bag | - | HPLC |
| Liquid/Precipit ation Stability | 20 mg/mL($H_2O$), 40 °C | Clear vial (closed) | 24 hours | HPLC |
| Solubility | Korean Pharmacopoeia 'Solubility' | | - | - |

**Experimental Example 1: Solubility Comparative Test**

The solubility of 9 types of the prepared tegoprazan acid addition salts was measured. The measurement was conducted according to the method described under "Solubility" in the General Notices of Appendix 1 of the 12th Edition of the Korean Pharmacopoeia. Each solubility was expressed by measuring the solubility of the acid addition salt itself, without conversion to the tegoprazan freebase.

[Table 4]

| Acid Addition Salt | Crystal line Form | Solubility (mg/mL) |
|---|---|---|
| Tegoprazan Freebase Crystalline Form A | Crystal line Form | < 1.0 |
| Hydrochloride Hydrate (Example 1) | Crystal line Form | 38.5 |
| Hydrobromide Salt (Comparative Example 1) | Crystal line | 10.0 |
| | Form | |
| Sulfate Salt (Comparative Example 4) | Crystal line Form | 666.7 |
| Hydrochloride Salt (Example 2) | Amorp hous Form | 40.8 |

(continued)

| Acid Addition Salt | Crystal line Form | Solubility (mg/mL) |
|---|---|---|
| Phosphate Salt (Comparative Example 5) | Amorp hous Form | 588.2 |
| Maleate Salt (Comparative Example 6) | Amorp hous Form | < 1.0 |
| Methanesulfonate Salt (Comparative Example 7) | Amorp hous Form | 909.1 |
| Methanesulfonate Salt (Comparative Example 8) | Crystal line Form | 1428.6 |
| Hemisalicylate Salt (Comparative Example 9) | Amorp hous Form | < 1.0 |

As may be seen in Table 4 above, among the 9 types of acid addition salts, 7 types of acid addition salts, excluding maleate salt and hemisalicylate salt, showed solubility at least 10 times or more compared to that of the commercially available tegoprazan freebase. In the case of maleate salt and hemisalicylate salt, solubility was shown to be 1.0 mg/mL or less, similar to the freebase of the compound of Formula 1. Therefore, tegoprazan hydrochloride salt (amorphous form) and hydrochloride hydrate crystalline form A are expected to show higher solubility compared to existing commercial free bases, which will be advantageous for drug absorption in the body.

**Experimental Example 2: Liquid Stability Comparative Test**

In the Experimental Example 1, a test for liquid stability was performed on acid addition salts (hydrochloride hydrate, sulfate salt, hydrochloride salt (amorphous form), phosphate salt, methanesulfonate salt (amorphous form), and methanesulfonate salt (crystalline form) showing an aqueous solubility of 30 mg/mL or more using the Comparative Examples 2 and 3 (malate salt and pidolate salt) as controls.

Each sample was dissolved in purified water to prepare a solution with a concentration of 20 mg/mL, stored at an external temperature of 40 °C, and analyzed by HPLC at the initial time point and after 24 hours to measure purity, and the change in the generated degradant (Formula 4) was measured. The analysis of total related substances and degradants was performed by taking a liquid from a solution prepared at a concentration of 20 mg/mL, diluting it to a concentration of 4 mg/mL with the diluent of the related substance test method (HPLC), and measuring it as a test solution, and analyzing it according to the related substance test method (HPLC). The results are shown in Table 5 below.

[Table 5]

| Acid Addition Salt | Initial | | After 24 hours | | Amount of Change | |
|---|---|---|---|---|---|---|
| | Purity (%) | Degradant (%) | Purity (%) | Degradant (%) | Purity (%) | Degradant (%) |
| Malate Salt | 99.98 | N.D. | Solid precipitated | | - | |
| Pidolate Salt | 99.98 | N.D. | Solid precipitated | | - | |
| Hydrochloride Hydrate | 99.97 | 0.012 | 99.50 | 0.428 | - 0.48 | 0.42 |
| Sulfate Salt | 99.89 | 0.080 | 93.95 | 5.414 | - 5.95 | 5.33 |
| Hydrochloride Salt (Amorphous Form) | 99.94 | 0.032 | 99.69 | 0.242 | - 0.25 | 0.21 |
| Phosphate Salt | 99.97 | 0.010 | Solid precipitated | | - | |
| Methanesulfonate Salt (Amorphous Form) | 99.90 | 0.063 | 99.34 | 0.552 | - 0.56 | 0.49 |
| Methanesulfonate Salt (Crystalline Form) | 99.95 | 0.028 | 99.47 | 0.446 | - 0.48 | 0.42 |

As a result of the test, it was confirmed that when stored at 40 °C for 24 hours, malate salt, pidolate salt, and phosphate salt showed a phenomenon of solid precipitation, while other acid addition salts maintained a homogeneous solution state. Additionally, it was confirmed that sulfate salt formed 5 % or more of degradants after 24 hours of storage and its purity decreased by about 6 %. In contrast, the 4 types of hydrochloride hydrate, hydrochloride salt, and methanesulfonate salt (amorphous form and crystalline form) were confirmed to have relatively significantly higher stability, with an increase in degradants being only 0.5 % or less.

**Experimental Example 3: Solid-state Stability Comparative Test**

A solid-state stability comparative test was conducted on each of the above-mentioned prepared tegoprazan acid addition salts. The stability comparative test was conducted for 1 month under the conditions shown in Table 6 below in accordance with The International Council for Harmonisation of Technical Requirements for Pharmaceuticals for Human Use (ICH) guideline, and the purity of the acid addition salt, total related substances, and the content of degradants were measured by HPLC at the initial time point and after 1 month. Total related substances and degradants were measured according to the related substance test method (HPLC).

[Table 6]

| Test Condition | Packaging Condition | Test Period |
|---|---|---|
| Accelerated Stability: 40 ± 2 °C / RH 75 ± 5 % | Double PE bag and Al bag | 1 Month |
| Stress Stability: Temperature (60 °C) | Amber Vial (closed) | |
| Stress Stability: Humidity (90 % RH) | Clear Vial (opened) | |

1) Accelerated Stability Test (40 ± 2 °C/RH 75 ± 5 %)

[Table 7]

| Acid Addition Salt | Initial (%) | | | After 1 Month (%) | | | Change (%) | | Remarks |
|---|---|---|---|---|---|---|---|---|---|
| | Purity | Total Related substances | Degradant | Purity | Total Related substances | Degradant | Purity | Degradant | |
| Malate Salt | 99.8 54 | 0.146 | 0.004 | 99.7 64 | 0.236 | 0.049 | - 0.09 0 | 0.045 | - |
| Pidolate Salt | 99.8 55 | 0.145 | 0.001 | 99.8 54 | 0.146 | 0.003 | - 0.00 1 | 0.002 | - |
| Hydrochloride Hydrate | 99.8 54 | 0.146 | 0.033 | 99.7 90 | 0.210 | 0.095 | - 0.06 4 | 0.062 | - |
| Hydrobromide Salt | 97.8 27 | 2.173 | 1.972 | 97.4 81 | 2.519 | 2.188 | - 0.34 6 | 0.216 | - |
| Sulfate Salt | 99.3 91 | 0.609 | 0.339 | 98.8 03 | 1.197 | 0.712 | - 0.58 8 | 0.373 | Discolorat ion |
| Hydrochloride Salt (Amorphous Form) | 99.7 93 | 0.207 | N.D. | 99.5 33 | 0.467 | 0.230 | - 0.26 0 | 0.230 | - |
| Phosphate | 99.7 | 0.254 | 0.085 | 99.6 | 0.327 | 0.205 | - | 0.120 | - |
| Salt | 46 | | | 73 | | | 0.07 3 | | |
| Maleate Salt | 99.6 43 | 0.357 | 0.027 | 99.3 21 | 0.679 | 0.083 | - 0.32 2 | 0.056 | - |
| Methanesulfo nate Salt (Amorphous Form) | 99.4 70 | 0.530 | 0.284 | 98.3 47 | 1.653 | 1.124 | - 1.12 3 | 0.840 | - |
| Methanesulfo nate Salt (Crystalline Form) | 99.7 35 | 0.265 | 0.122 | 99.2 73 | 0.727 | 0.518 | - 0.46 2 | 0.396 | - |
| Hemisalicylate Salt | 99.7 92 | 0.208 | 0.001 | 99.7 92 | 0.208 | 0.004 | - | 0.003 | - |

According to the accelerated stability test results in Table 7 above, the malate salt, pidolate salt, hydrochloride hydrate, and hemisalicylate salt showed a purity change of 0.1 % or less after 1 month, indicating that they were stable. In contrast, hydrobromide salt, sulfate salt, maleate salt, methanesulfonate salt (amorphous form), and methanesulfonate salt (crystalline form) showed an increase of 0.3 % or more in total related substances after 1 month, and in the case of sulfate salt, a change in appearance of the solid occurred, such as discoloration. When powder X-ray diffraction (PXRD) analysis was performed on all acid addition salts at the initial time point and after 1 month, no polymorphic transition was confirmed.

2) Stress Stability Test (temperature 60 °C)

[Table 8]

| Acid Addition Salt | Initial (%) | | | After 1 Month (%) | | | Change (%) | | Remarks |
|---|---|---|---|---|---|---|---|---|---|
| | Purity | Total Related substances | Degradant | Purity | Total Related substances | Degradant | Purity | Degradant | |
| Malate Salt | 99.8 54 | 0.146 | 0.004 | 97.9 74 | 2.026 | 1.320 | - 1.88 0 | 1.316 | - |
| Pidolate Salt | 99.8 55 | 0.145 | 0.001 | 99.6 47 | 0.353 | 0.088 | - 0.20 8 | 0.087 | - |
| Hydrochloride Hydrate | 99.8 54 | 0.146 | 0.033 | 97.4 95 | 2.505 | 1.779 | - 2.35 9 | 1.746 | - |
| Hydrobromide Salt | 97.8 27 | 2.173 | 1.972 | 91.0 95 | 8.905 | 7.787 | - 6.73 2 | 5.815 | Discolorat ion |
| Sulfate Salt | 99.3 91 | 0.609 | 0.339 | 96.5 59 | 3.441 | 2.345 | - 2.83 2 | 2.006 | Discolorat ion |
| Hydrochloride Salt (Amorphous Form) | 99.7 93 | 0.207 | N.D. | 98.4 64 | 1.536 | 1.006 | - 1.32 9 | 1.006 | - |
| Phosphate Salt | 99.7 46 | 0.254 | 0.085 | 97.5 77 | 2.423 | 1.668 | - 2.16 9 | 1.583 | - |
| Maleate Salt | 99.6 43 | 0.357 | 0.027 | 93.9 30 | 6.070 | 1.248 | - 5.71 3 | 1.221 | - |
| Methanesulfo nate Salt (Amorphous Form) | 99.4 70 | 0.530 | 0.284 | 87.7 55 | 12.245 | 9.245 | - 11.7 15 | 8.961 | Discolorat ion |
| Methanesulfo nate Salt (Crystalline Form) | 99.7 35 | 0.265 | 0.122 | 94.5 92 | 5.408 | 4.167 | - 5.14 3 | 4.045 | Discolorat ion |
| Hemisalicylate Salt | 99.7 92 | 0.208 | 0.001 | 99.7 32 | 0.268 | 0.039 | - 0.06 0 | 0.038 | - |

According to the results of the stress stability test (temperature 60 °C) in Table 8 above, the total related substances of hydrobromide salt, maleate salt, methanesulfonate salt (amorphous form), and methanesulfonate salt (crystalline form) increased by 5.0 % or more after 1 month, and it was confirmed that the degradant of Formula 4 also increased significantly. Moreover, in the case of hydrobromide salt, sulfate salt, methanesulfonate salt (amorphous form) and methanesulfonate salt (crystalline form), showed a change in appearance, a change in the color of the solid. In contrast, malate salt, pidolate salt, hydrochloride hydrate, and hemisalicylate salt were confirmed to be stable with significantly lower total related substances and degradants of Formula 4 after 1 month.When powder X-ray diffraction (PXRD) analysis was performed on all acid addition salts at the initial time point and after 1 month, no polymorphic transition was confirmed.

3) Stress Stability (humidity 90 % RH)

[Table 9]

| Acid Addition Salt | Initial (%) | | | After 1 Month (%) | | | Change (%) | | Remarks |
|---|---|---|---|---|---|---|---|---|---|
| | Purity | Total Related substances | Degradant | Purity | Total Related substances | Degradant | Purity | Degradant | |
| Malate Salt | 99.8 54 | 0.146 | 0.004 | - | - | - | - | - | Melted |
| Pidolate Salt | 99.8 55 | 0.145 | 0.001 | - | - | - | - | - | Melted |
| Hydrochloride Hydrate | 99.8 54 | 0.146 | 0.033 | 99.7 90 | 0.210 | 0.086 | - 0.06 4 | 0.053 | |
| Hydrobromide Salt | 97.8 27 | 2.173 | 1.972 | 96.0 78 | 3.922 | 3.698 | - 1.74 9 | 1.726 | |
| Sulfate Salt | 99.3 91 | 0.609 | 0.339 | 83.3 63 | 16.637 | 14.500 | - 16.0 28 | 14.161 | Polymorp hic Transition |
| Hydrochloride Salt (Amorphous Form) | 99.7 93 | 0.207 | N.D. | 99.6 65 | 0.335 | 0.133 | - 0.12 8 | 0.133 | |
| Phosphate Salt | 99.7 46 | 0.254 | 0.085 | - | - | - | - | - | Melted |
| Maleate Salt | 99.6 43 | 0.357 | 0.027 | - | - | - | - | - | Melted |
| Methanesulfo nate Salt (Amorphous Form) | 99.4 70 | 0.530 | 0.284 | - | - | - | - | - | Melted |
| Methanesulfo nate Salt (Crystalline Form) | 99.7 35 | 0.265 | 0.122 | - | - | - | - | - | Melted |
| Hemisalicylate Salt | 99.7 92 | 0.208 | 0.001 | 98.2 41 | 1.759 | N.D. | - 1.55 1 | - 0.001 | |

According to the results of the stress stability test (humidity 90 % RH) in Table 9 above, the hydrochloride hydrate was found to be the most stable with a change of 0.1 % or less, followed by the hydrochloride salt amorphous form with a low change of 0.15 % or less. In contrast, malate salt, pidolate salt, phosphate salt, maleate salt, methanesulfonate salt (amorphous form), and methanesulfonate salt (crystalline form) were observed to absorb moisture and deliquesce within two days of the test initiation, confirming their very low stability to humidity.When powder X-ray diffraction (PXRD) analysis was performed on 5 types of acid addition salts that maintained a solid state after 1 month, it was confirmed that polymorphic transition occurred in the case of sulfate salt, as the initial crystalline form and the new crystalline form existed in a mixed form (see FIG. 7C).

**Experimental Example 4: Photostability Comparative Test**

A photostability comparative test was conducted on each of the above-mentioned prepared tegoprazan acid addition salts. The test was conducted by exposing the sample to light with an energy of 200 W hr/m$^2$ of ultraviolet light and 1.2 Mlux hr of visible light in accordance with The International Council for Harmonisation of Technical Requirements for Pharmaceuticals for Human Use (ICH) guideline, with the samples packaged in a single PE bag and spread thinly enough to ensure exposure to the light source, taking humidity stability into consideration. The purity of the acid addition salts and the change in the amount of degradants at the initial time point and after exposure to ultraviolet light or visible light were measured by HPLC.

1) Ultraviolet Light (200 W-hr/m$^2$) Exposure Test

[Table 10]

| Acid Addition Salt | Initial (%) | | | After Ultraviolet Light Exposure (200 W hr/m2)(%) | | | Change (%) | | Remarks |
|---|---|---|---|---|---|---|---|---|---|
| | Purit y | Total Related substan ces | Degrad ant | Purit y | Total Related substan ces | Degrad ant | Puri ty | Degrad ant | |
| Malate Salt | 99.8 54 | 0.146 | 0.004 | 99.8 28 | 0.172 | 0.011 | 0.02 6 | 0.007 | |
| Pidolate Salt | 99.8 55 | 0.145 | 0.001 | 99.7 34 | 0.266 | 0.020 | 0.12 1 | 0.019 | |
| Hydrochloride Hydrate | 99.8 54 | 0.146 | 0.033 | 99.9 10 | 0.090 | 0.043 | 0.05 6 | 0.010 | |
| Hydrobromid e Salt | 97.8 27 | 2.173 | 1.972 | 97.7 35 | 2.265 | 2.028 | 0.09 2 | 0.056 | |
| Sulfate Salt | 99.3 91 | 0.609 | 0.339 | 98.2 52 | 1.748 | 1.415 | 1.13 9 | 1.076 | |
| Hydrochloride Salt (Amorphous Form) | 99.7 93 | 0.207 | N.D. | 99.6 87 | 0.313 | 0.142 | - 0.10 6 | 0.142 | |
| Phosphate Salt | 99.7 46 | 0.254 | 0.085 | 99.7 23 | 0.277 | 0.096 | 0.02 3 | 0.011 | |
| Maleate Salt | 99.6 43 | 0.357 | 0.027 | 99.6 46 | 0.354 | 0.032 | 0.00 3 | 0.005 | |
| Methanesulfo nate Salt (Amorphous Form) | 99.4 70 | 0.530 | 0.284 | 99.4 01 | 0.599 | 0.366 | - 0.06 9 | 0.082 | |
| Methanesulfo nate Salt (Crystalline Form) | 99.7 35 | 0.265 | 0.122 | 99.6 61 | 0.339 | 0.168 | - 0.07 4 | 0.046 | |

(continued)

| Acid Addition Salt | Initial (%) | | | After Ultraviolet Light Exposure (200 W hr/m2)(%) | | | Change (%) | | Remarks |
|---|---|---|---|---|---|---|---|---|---|
| | Purit y | Total Related substan ces | Degrad ant | Purit y | Total Related substan ces | Degrad ant | Puri ty | Degrad ant | |
| Hemisalicylat e Salt | 99.7 92 | 0.208 | 0.001 | 98.6 40 | 1.360 | 0.360 | 1.15 2 | 0.359 | Discolorat ion |

2) Visible Light (1.2 Mlux-hr) Exposure Test

[Table 11]

| Acid Addition Salt | Initial (%) | | | After Ultraviolet Light Exposure (200 W hr/m2)(%) | | | Change (%) | | Remarks |
|---|---|---|---|---|---|---|---|---|---|
| | Purit y | Total Related substan ces | Degrad ant | Purit y | Total Related substan ces | Degrad ant | Puri ty | Degrad ant | |
| Malate Salt | 99.8 54 | 0.146 | 0.004 | 99.7 37 | 0.263 | 0.035 | 0.11 7 | 0.031 | |
| Pidolate Salt | 99.8 55 | 0.145 | 0.001 | 99.5 37 | 0.463 | 0.068 | 0.31 8 | 0.067 | |
| Hydrochloride Hydrate | 99.8 54 | 0.146 | 0.033 | 99.7 74 | 0.226 | 0.046 | 0.08 0 | 0.013 | |
| Hydrobromid e Salt | 97.8 27 | 2.173 | 1.972 | 97.7 73 | 2.227 | 1.956 | 0.05 4 | - 0.016 | Discolorat ion |
| Sulfate Salt | 99.3 | 0.609 | 0.339 | 97.2 | 2.754 | 2.273 | - | 1.934 | |
| | 91 | | | 46 | | | 2.14 5 | | |
| Hydrochloride Salt (Amorphous Form) | 99.7 93 | 0.207 | N.D. | 99.3 63 | 0.637 | 0.207 | 0.43 0 | 0.207 | |
| Phosphate Salt | 99.7 46 | 0.254 | 0.085 | 99.5 51 | 0.449 | 0.140 | 0.19 5 | 0.055 | |
| Maleate Salt | 99.6 43 | 0.357 | 0.027 | 99.4 92 | 0.508 | 0.033 | 0.15 1 | 0.006 | |
| Methanesulfo nate Salt (Amorphous Form) | 99.4 70 | 0.530 | 0.284 | 99.0 91 | 0.909 | 0.438 | -0.37 9 | 0.154 | |
| Methanesulfo nate Salt (Crystalline Form) | 99.7 35 | 0.265 | 0.122 | 99.5 88 | 0.412 | 0.208 | -0.14 7 | 0.086 | |
| Hemisalicylat e Salt | 99.7 92 | 0.208 | 0.001 | 98.9 79 | 1.021 | 0.245 | 0.81 3 | 0.244 | Discolorat ion |

According to the results of the photostability test in Tables 10 and 11 above, when examining the changes in purity and degradants after exposure to ultraviolet light and visible light of the acid addition salt, the hydrochloride hydrate was confirmed to be the most stable, with degradants of 0.1 % or less for both light sources. On the other hand, hydrobromide salt showed changes in appearance under visible light exposure conditions, and hemisalicylate salt showed changes in

appearance under ultraviolet light and visible light exposure conditions. In summary of the results of the liquid stability test and solid stability test (Table 12), it was confirmed that hydrochloride hydrate and hydrochloride salt (amorphous form) were acid addition salts that were resistant to humidity and had high stability overall, and among them, hydrochloride hydrate was particularly confirmed to have significantly excellent stability.

[Table 12]

| Acid Addition Salt | Solubility | Change in Quality (%) | | | | | | | | | |
|---|---|---|---|---|---|---|---|---|---|---|---|
| | | Accelerated (4 weeks) | | 60 °C (4 weeks) | | 90 % RH (4 weeks) | | Light (Ultraviolet) | | Light (Visible) | |
| | | Purity | Degradant | Purity | Degradant | Purity | Degradant | Purit y | Degradant | Purit y | Degradant |
| Malate Salt | 714.3 | -0.090 | 0.045 | -1.880 | 1.316 | Melted | | 0.026 | 0.007 | 0.11 7 | 0.031 |
| Pidolate Salt | 666.7 | -0.001 | 0.002 | -0.208 | 0.087 | Melted | | 0.121 | 0.019 | 0.31 8 | 0.067 |
| **Hydrochloride Hydrate** | **38.5** | **-0.064** | **0.062** | **-2.359** | **1.746** | **- 0.064** | **0.053** | **0.056** | **0.010** | **- 0.08 0** | **0.013** |
| **Hydrochloride Salt (Amorphous Form)** | **40.8** | **-0.260** | **0.230** | **-1.329** | **1.006** | **- 0.128** | **0.133** | **- 0.106** | **0.142** | **- 0.43 0** | **0.207** |
| Methanesulfonate Salt (Amorphous Form) | 909.1 | -1.123 | 0.840 | 11.715 | 8.961 | Melted | | 0.069 | 0.082 | 0.37 9 | 0.154 |
| Methanesulfonate Salt (Crystalline Form) | 1428. 6 | -0.462 | 0.396 | -5.143 (Discoloration) | 4.045 | Melted | | 0.074 | 0.046 | 0.14 7 | 0.086 |

**Experimental Example 5. Powder X-ray Diffraction (PXRD) Analysis**

PXRD analysis was performed to analyze the crystallinity change of each of the above-mentioned prepared tegoprazan acid addition salts. In the solid-state stability (accelerated stability, stress stability (60 °C stress stability (90 % RH)) of the Experimental Example 3, PXRD analysis of each acid addition salt was performed at the initial stage and after 1 month of storage in the test. PXRD analysis was performed according to the PXRD test method.

As a result, it was confirmed that no polymorphic transition was observed under any other conditions except for the case of tegoprazan sulfate salt after 1 month under stress stability conditions (90 % RH) (see FIG. 7C).

**Experimental Example 6. Temperature Differential Scanning Calorimetry (DSC) Analysis**

DSC analysis was performed on each of the above-mentioned prepared tegoprazan acid addition salts. DSC analysis was performed using a temperature differential scanning calorimeter (SCINCO DSC N-650) in a sealed pan, at a scan rate of 10 °C/min from 25 °C to 400 °C under nitrogen purge.

As a result, it was confirmed that each acid addition salt was well synthesized from the freebase of Formula 1 compound in crystalline form (see FIG. 1B). DSC analysis was performed according to the DSC test method.

**Conclusion**

It was confirmed that tegoprazan hydrochloride hydrate and hydrochloride salt have a higher solubility at least 10 times or more compared to that of the commercially available tegoprazan freebase, and thus expected to achieve sufficient absorption upon in vivo administration. In addition, as a result of the liquid stability test, it was confirmed that the hydrochloride hydrate and hydrochloride salt had significantly better stability compared to other acid addition salts, with the increase in degradants being only 0.5 % or less, and as a result of the solid stability test, it was confirmed that the hydrochloride hydrate had significantly better stability compared to other acid addition salts throughout the accelerated stability test and the stress stability test. In addition, the hydrochloride hydrate showed significantly better stability

compared to other acid addition salts in photostability tests. Additionally, it was confirmed that the hydrochloride salt has overall superior liquid stability, solid stability, and photostability compared to other acid addition salts.

Based on the liquid stability test results, methanesulfonate (amorphous and crystalline) proved to be very stable, with an increase in degradation products of only 0.5% or less. However, the solid-state stability test revealed that the content of total related substances and degradants significantly increased, or the substance deliquesced due to moisture absorption, confirming its low stability. Although the previously disclosed maleate and pidolate salts exhibited high solubility, the **liquid stability test** revealed that a solid **precipitated** after 24 hours. Under the stress conditions of a 90% RH solid-state stability test, the salts deliquesced within 24 hours and became unstable. Furthermore, in the photostability test, a large amount of total related substances and degradants were generated compared to tegoprazan hydrochloride hydrate, rendering them unstable. In contrast, the hydrochloride salt and hydrochloride hydrate did not show phenomena such as solid precipitation or deliquescence under the same conditions, and the degradants were also generated in a significantly low content, confirming them stable. Therefore, the stability test results showed that tegoprazan hydrochloride salt and hydrochloride hydrate showed significantly superior stability overall compared to other acid addition salts. In particular, it was confirmed that tegoprazan hydrochloride hydrate showed significantly better stability compared to other acid addition salts, and also had significantly better stability compared to the previously disclosed malate salt and pidolate salt. In addition, based on a comprehensive comparison of solubility, liquid stability, solid-state stability, and photostability, it may be said that the hydrochloride hydrate and the hydrochloride salt (amorphous form) are pharmaceutically excellent acid addition salts that are easy to store as raw pharmaceutical materials and have stability even after being prepared into pharmaceuticals.

**Experimental Example 7. Comparative Test for Formulation**

The related substances analytical methods performed in Examples 7-1 to 7-3 are as follows.

<Related substance Test Method (HPLC)>

The amount of total related substances under stress storage conditions (temperature/humidity) was measured using liquid chromatography (HPLC) using an Infinity 1260 (Agilent) equipment. The analysis conditions are as follows.

[Solution Preparation]

Blank Solution (Diluent)

TFA: ACN: Water were mixed in a volume ratio of 1:70:30.

**[Device Conditions]**

| | | | |
|---|---|---|---|
| Detector | Ultraviolet absorbance spectrophotometer (measurement wavelength: 220 nm) | | |
| Column | C18 (4.6 mm X 250 mm, 5 µm) or a similar column | | |
| Column Temperature | 30 °C | | |
| Mobile Phase A | 0.1 % $H_3PO_4$ solution | | |
| Mobile Phase B | ACN | | |
| Gradient Condition | Time (Minutes) | Mobile Phase A (%) | Mobile Phase B (%) |
| | 0.0 | 85 | 15 |
| | 12.0 | 76 | 24 |
| | 28.0 | 5 | 95 |
| | 32.0 | 5 | 95 |
| | 32.01 | 85 | 15 |
| | 40.0 | 85 | 15 |
| Flow rate | 0.8 mL/min | | |

(continued)

| | |
|---|---|
| Injection Volume | 5 μL |
| Analysis Time | 40 minutes |

Operation and Calculation: The test was conducted according to the liquid chromatography method with the concentration of the standard solution and test solution set to 0.2 mg/mL, and the amount of related substances was calculated using the following equation.

$$\text{Amount of each related substance (\%)} = \frac{A_T}{A_S} \times \frac{C_S}{C_T} \times 100$$

$A_T$ = Area of each related substance peak obtained from the test solution
$A_S$ = Area of the tegoprazan peak obtained from the standard solution
$C_S$ = Concentration of standard solution (mg/mL)
$C_T$ = Concentration of test solution (mg/mL)

The amount of total related substances are calculated as the sum of the amounts of individual related substances.

**Experimental Example 7-1. Raw Material Stability Test (Comparative Test with Tegoprazan Freebase)**

A comparative test was conducted on the tegoprazan hydrochloride hydrate prepared in the Example 1 with the tegoprazan freebase crystalline form A contained in the reference drug (K-CAB tablet 50 mg and K-CAB orally disintegrating tablet 50 mg) disclosed in the Ministry of Food and Drug Safety Pharmaceutical Integrated Information System, and the product obtained in the Preparation Example 1 was used. For further comparison, the crystalline forms of sulfate salt and methanesulfonate salt prepared in the comparative examples, which were confirmed to have high solubility as acid addition salts, were used.

To compare the stability of the main ingredient raw materials themselves, the degree of change in appearance and generation of related substances under stress conditions were compared. Stability tests were conducted under each of the stress temperature condition and stress humidity condition shown in Table 13 below.

[Table 13]

| No. | Main ingredient | | Stress Conditions |
|---|---|---|---|
| C01 | Tegoprazan | Freebase | - Temperature (60 °C)<br>- Humidity (90 % RH) |
| C02 | | Hydrochloride Hydrate | |
| C03 | | Sulfate Salt | |
| C20 | | Methanesulfonate Salt (Crystalline Form) | |

(1) Appearance Observation

The change in appearance of tegoprazan freebase and tegoprazan acid addition salts were observed after 3 weeks under initial conditions and stress storage conditions (temperature/humidity). The results are shown in Table 14 below.

[Table 14]

| Tegoprazan | Initial | 60 °C | 90 % RH |
|---|---|---|---|
| Freebase | No change in appearance | No change in appearance | No change in appearance |
| Hydrochloride Hydrate | No change in appearance | No change in appearance | No change in appearance |
| Sulfate Salt | Pungent odor | Pungent odor | Pungent odor |

(continued)

| Tegoprazan | Initial | 60 °C | 90 % RH |
|---|---|---|---|
| Methanesulfonate Salt (Crystalline Form) | No change in appearance | No change in appearance | Dissolved to transparency |

As shown in Table 14 above, the sulfate salt had a persistent, pungent odor from the initial raw material itself due to the characteristic of the acid. In the case of methanesulfonate salt, significant moisture absorption of the raw material was observed under stress humidity conditions, leading to the raw material's deliquescence. Therefore, to secure stability and excellent sensory properties when developing orally disintegrating tablets, it was confirmed that hydrochloride hydrate would be the most suitable as an acid addition salt. According to the results of FIG. 13, when the amount of total related substances generated under stress temperature/humidity storage conditions was confirmed, the amount of related substances generated was significantly increased in tegoprazan sulfate salt and methanesulfonate salt (crystalline form) compared to tegoprazan freebase, whereas tegoprazan hydrochloride hydrate showed a similar amount of related substances generated as tegoprazan freebase, confirming its stability.

**Experimental Example 7-2**. **Combination Compatibility Test**

A compatibility study was conducted to confirm the interaction of excipients with tegoprazan acid addition salts and their effect on stability. The study evaluated excipients based on their potential use for various formulation purposes. To confirm the influence of excipients used in the actual reference drugs, the excipients used in the reference drugs (K-CAB tablets 50 mg and K-CAB orally disintegrating tablets 50 mg) disclosed in the Ministry of Food and Drug Safety Pharmaceutical Integrated Information System were selected as candidates, and the other excipients were excipients commonly used in tablet and orally disintegrating tablet formulations. The design of the combination compatibility test is described in Table 15 below. The stability test was conducted by storing the product for 1 week and 3 weeks, respectively, under the storage conditions of 60 °C, which is a stress temperature condition, and 90 % RH, which is a stress humidity condition, and measuring the amount of total related substances. The results are shown in FIGS. 14 to 16. The results of tegoprazan hydrochloride hydrate are shown in FIG. 14, the results of sulfate salt are shown in FIG. 15, and the results of methanesulfonate salt (crystalline form) are shown in FIG. 16, and for convenience, the combined samples are indicated as -1, -2, -3, respectively.

[Table 15]

| No. | Ingredient | | Stress Conditions | Analytical Method |
|---|---|---|---|---|
| C04 | Diluent | Microcrystalline cellulose | Temperature (60 °C) or Humidity (90 % RH) | HPLC |
| C05 | | Lactose hydrate | | |
| C06 | | Lactose anhydrous | | |
| C07 | | D-mannitol | | |
| C*01 | | Pearlitol flash(mannitol+starch) | | |
| C08 | Disintegrant | L-HPC | | |
| C09 | | Crospovidone | | |
| C10 | | Croscarmellose sodium | | |
| C*02 | | Corn starch | | |
| C*03 | | Crospovidone(SF) | | |
| C12 | Binder | Copovidone | | |
| C13 | | HPC | | |
| C14 | Lubricant | SiO2 | | |
| C15 | | Talc | | |
| C16 | | Sodium stearyl fumarate | | |
| C17 | | Magnesium stearate | | |
| C18 | Coating | OPADRY 85F | | |

(continued)

| No. | Ingredient | | Stress Conditions | Analytical Method |
|---|---|---|---|---|
| C19 | | OPADRY 03B | | |
| C*04 | sweetener | Maltitol | | |
| C*05 | | Sucralose | | |
| C*06 | | Stevia glycosides | | |
| C*07 | | Acesulfame potassium | | |
| C*08 | | Xylitol | | |
| C*09 | | Isomalt | | |
| C*10 | Flavor | Mint | | |
| C*11 | | Drink flavor powder (Bacchus type) | | |
| C*12 | | Green Grape Mint | | |
| C*13 | | Eucalyptus | | |

As a result of checking the combination compatibility between tegoprazan acid addition salts and additives under stress temperature/humidity storage conditions, it was confirmed that in the hydrochloride hydrate combination, only C12 (Copovidone), used as a binder in tablets, and C*07 (Acesulfame potassium), used as a sweetener in orally disintegrating tablets, showed an increase in the amount of related substances, resulting in poor combination compatibility. However, since the amount of generated related substances increased from the beginning even when the combination was mixed with other acid addition salts, it is determined that the effect was greater when a peak derived from the excipient itself was detected at the HPLC analysis wavelength, rather than an increase in related substances caused by interaction with the main ingredient. On the other hand, it can be confirmed that the amount of total related substances generated in other additive combinations is significantly improved in the combination of tegoprazan hydrochloride hydrate compared to the combination of tegoprazan sulfate salt and methanesulfonate salt (crystalline form). Therefore, it was confirmed that tegoprazan hydrochloride hydrate has minimal interaction with mixed additives and may secure significantly superior stability compared to other acid addition salts when formulated as a tablet or orally disintegrating tablet.

**Experimental Example 7-3. Stability Test by Formulation**

To confirm the stability according to the formulation, tablets and orally disintegrating tablets with different amounts of tegoprazan acid addition salt were prepared and the change in appearance and the degree of generation of related substances under stress conditions were compared. To minimize the influence of additives, each test formulation was selected with the same additives as the reference drug (K-CAB tablets 50 mg, K-CAB orally disintegrating tablets 50 mg) disclosed in the Ministry of Food and Drug Safety Pharmaceutical Integrated Information System, and to minimize differences due to the granule manufacturing process, the formulation was prepared using the simple mixing (direct compression) method. Each formulation was as shown in Tables 16 and 17 below, and the stability conditions were conducted for each formulation under the storage conditions of 60 °C, which is a stress temperature condition, and 90 % RH, which is a stress humidity condition, respectively.

[Table 16]

| Ingredients and Amounts [Tablet] | |
|---|---|
| Tegoprazan | 50 mg (50 mg as freebase) |
| Hydroxypropyl cellulose | 6 mg |
| D-mannitol | 50 mg |
| Microcrystalline cellulose | 80 mg |
| Croscarmellose sodium | 10 mg |
| Colloidal silicon dioxide | 2 mg |
| Magnesium stearate | 2 mg |

(continued)

| Ingredients and Amounts [Tablet] | |
|---|---|
| Tegoprazan | 50 mg (50 mg as freebase) |
| Opadry Pink | 6 mg |

[Table 17]

| Ingredients and Amounts [Orally Disintegrating Tablet] | |
|---|---|
| Tegoprazan | 50 mg (50 mg as freebase) |
| D-mannitol | 50 mg |
| Sucralose | 3.5 mg |
| Pearlitol Flash | 192.3 mg |
| Crospovidone | 17.5 mg |
| Maltitol | 17.5 mg |
| Enzyme-treated stevia | 1.8 mg |
| Peppermint | 3.5 mg |
| Colloidal silicon dioxide | 3.5 mg |
| Magnesium stearate | 10.5 mg |

(1) Appearance Observation

The change in appearance of the reference drug (K-CAB tablets 50 mg and K-CAB orally disintegrating tablets 50 mg) and tablets and orally disintegrating tablets prepared with different tegoprazan acid addition salts were confirmed after being stored under stress storage conditions (60 °C/90 % RH) for 3 weeks, and is shown in FIG. 17.

According to FIG. 17, it can be confirmed that the weight increases regardless of the formulation type or the type of acid addition salt under stress humidity conditions. The degree of weight gain was confirmed to be greater in the sulfate salt and methanesulfonate salt (crystalline form) compared to the reference drug, whereas in the case of the hydrochloride hydrate, it was confirmed to be similar to the reference drug, and in the tablet formulation, it was observed to be smaller. Therefore, the hydrochloride hydrate is expected to be advantageous for securing formulation stability by minimizing the degree of moisture absorption even under high humidity.

(2) Analysis of Related substances

Stability tests were conducted under stress storage conditions (temperature/humidity) for each formulation. The results of measuring the amount of total related substances after storage under stress conditions for 1 week or 3 weeks are shown in FIG. 18 and FIG. 19.

According to FIGS. 18 and 19, when the amount of related substances generated was confirmed under each of the stress storage conditions of temperature and humidity, the amount of total related substances generated in both formulations was significantly increased compared to the reference drug when prepared with sulfate salt and methanesulfonate salt (crystalline form), whereas when prepared with hydrochloride hydrate, both formulations showed a similar amount of related substances generated to the reference drug, indicating that hydrochloride hydrate showed significantly superior stability when formulated compared to other acid addition salts.

**Experimental Example 7-4. Disintegration Time Evaluation**

Orally disintegrating tablets are a convenient formulation for patients with impaired swallowing ability, as they disintegrate in the oral cavity even taken without water, and therefore require rapid disintegration in the oral cavity. Therefore, to confirm the disintegration time, 18 mL of a pH 6.8 saline solution, warmed to about 37 °C that is most similar to saliva, was placed on a petri dish, and the orally disintegrating tablet prepared in Experimental Example 7-3 was placed thereon, and the time it took for the entire surface of the orally disintegrating tablet to become wet with the solution (wetting time) was measured, and the results are shown in FIG. 20.

According to the results of FIG. 20, the hydrochloride hydrate was confirmed to absorb the solution the fastest,

and the absorption time was significantly shorter even compared to commercially available orally disintegrating tablets. Therefore, when formulated as an orally disintegrating tablet, hydrochloride hydrate showed the fastest disintegration, and is expected to provide improved ease of administration and a rapid onset of action.

**Experimental Example 7-5. Evaluation of Dissolution Rate Under Acidic Conditions**

In the case of the reference drug (K-CAB Tablet 50 mg), it is known to show rapid drug release in the gastric fluid environment of the stomach upon administration. Accordingly, a comparative test of the drug dissolution rate was conducted between the control drug (K-CAB 50 mg tablet) and the test drug (tegoprazan hydrochloride hydrate tablet) prepared in said Experimental Example 7-3, under acidic conditions that mimic the gastric fluid environment.

The dissolution test was conducted according to the Dissolution Test Method 2 (Paddle Method) of the General Test Methods of the 12th edition of the Korean Pharmacopoeia, as shown in Table 18 below. The results are shown in FIG. 21.

[Table 18]

| | |
|---|---|
| Apparatus | Dissolution Test Method 2 (Paddle Method) of the Korean Pharmacopoeia |
| Test Solution | pH 1.2, pH 4.0 |
| Agitation Speed | 50 rpm |
| Volume of Test Solution | 900 mL |
| Agitation Temperature | 37±0.5 °C |

According to the results of FIG. 21, both tegoprazan hydrochloride hydrate tablets and the reference drug, K-CAB tablets 50 mg, showed immediate-release characteristics of 85 % within 15 minutes without release delay at pH 1.2. In particular, tegoprazan hydrochloride hydrate tablets showed 85 % release within 15 minutes under both pH 1.2 and pH 4.0 conditions. In contrast, the reference drug, K-CAB tablet, showed an immediate-release characteristic of 85 % within 15 minutes at pH 1.2, but the release was somewhat delayed at pH 4.0, reaching 85 % release after 15 minutes.

**Experimental Example 7-6. Dissolution Test Under Gastrointestinal Environment**

In general, food may alter the bioavailability of drugs by delaying gastric emptying time, changing the acidity of the gastrointestinal tract and the like. In the post-prandial state, the gastrointestinal pH increases, which may affect the dissolution rate of the drug, and there is a possibility that the active ingredient may pass into the intestinal environment rather than the gastric fluid environment, leading to its dissolution, due to the delay in the release of the active ingredient from the stomach. Therefore, a dissolution test was performed under intestinal fluid conditions rather than gastric fluid conditions.

A dissolution test was performed on the commercially available K-CAB tablets (reference drug) 50 mg and the test drug (tegoprazan hydrochloride hydrate tablets) prepared in the Experimental Example 7-3 containing the same dose of tegoprazan. The dissolution test solution was selected as a FaSSiF solution (pH 6.5) simulating fasting artificial intestinal fluid with pH 6.8, and the dissolution test was conducted according to the Dissolution Test Method 2 (Paddle Method) of the General Test Methods of the 12th Edition of the Korean Pharmacopoeia, as shown in Table 19 below. The results are shown in FIG. 22.

[Table 19]

| | |
|---|---|
| Apparatus | Dissolution Test Method 2 (Paddle Method) of the Korean Pharmacopoeia |
| Test Solution | pH 6.8, FaSSiF |
| Agitation Speed | 50 rpm |
| Volume of Test Solution | 900 mL |
| Agitation Temperature | 37±0.5 °C |

As a result of the test, the dissolution rate of tegoprazan hydrochloride hydrate tablets was high, 85 % or more within 15 minutes in both pH 6.8 and FaSSiF solution, while the reference drug (K-CAB tablets) showed a relatively significantly lower dissolution rate.

**It** is known that the $T_{max}$ of previously known tegoprazan freebase is delayed by up to 8 hours when administered

after a meal compared to when administered before a meal (Clin Transl Sci. 2021;14:934-941). This may be explained by differences in drug absorption profiles due to differences in solubility caused by changes in pH before and after meals. In contrast, in the case of tegoprazan hydrochloride hydrate, the solubility is at least 10 times or more higher compared to that of tegoprazan freebase when measured according to the method described in the 'Solubility' section of the General Test Methods of the Korean Pharmacopoeia (see Experimental Example 1 above), and it is believed that this high solubility may minimize changes in dissolution due to changes in pH. Therefore, it is expected that tegoprazan hydrochloride hydrate may minimize the effect of food on drug absorption compared to conventional commercial K-CAB tablets and secure high bioavailability, in other words, stable high bioavailability, regardless of food intake. Additionally, tegoprazan hydrochloride hydrate has significantly improved ease of administration in that it may be administered regardless of diet.

**Experimental Example 7-7. Pharmacokinetic Absorption Evaluation in Beagle Dogs**

To evaluate the pharmacokinetic characteristics of tegoprazan hydrochloride hydrate, a pharmacokinetic test was performed on commercially available K-CAB tablets (reference drug) 50 mg and the test drug (tegoprazan hydrochloride hydrate tablet) prepared in Experimental Example 7-3 containing the same dose of tegoprazan.

A total of 18 male beagle dogs (17 to 20 months old) were tested, 9 dogs per test group, in a crossover design under fasting conditions, and the test was orally administered after fasting for 16 hours or more the day before administration.

One tablet of each of the test drug and reference drug was orally administered, and blood samples were collected before administration (0 hour) and at 0.25, 0.5, 0.75, 1, 1.5, 2, 3, 4, 6, 8, 12, and 24 hours after administration. 2 mL of blood was collected from the jugular vein, immediately centrifuged to separate plasma, and stored in a freezer until analysis. LC-MS/MS was used for the analysis of tegoprazan in plasma, and Tegoprazan-d6 was used as the internal standard substance. The results are shown in Table 20 and FIG. 23 below.

[Table 20]

| Variable | Test drug (T) | Reference drug (R) | T/R ratio(%) |
|---|---|---|---|
| $AUC_{last}$ (ng·hr/mL) | 18180.80 | 19210.80 | 94.64 |
| $C_{max}$ (ng/mL) | 4342.13 | 4588.41 | 94.63 |
| $T_{max}$(hr) | 0.5 | 0.75 | |

According to the results of Table 20 and FIG. 23 above, the test drug secured a faster $T_{max}$ compared to the reference drug, and the maximum drug concentration ($C_{max}$) and the area under the blood concentration-time curve (AUC) after drug administration showed similar results. This suggests that under fasting conditions, the test drug achieves a bioavailability (degree of systemic exposure) comparable to the reference drug, while its improved solubility shortens $T_{max}$, which is expected to result in a more rapid onset of action.

## Claims

**1.** A salt, which is a hydrochloride or hydrochloride hydrate of a compound of Formula 1 below:

[Formula 1]

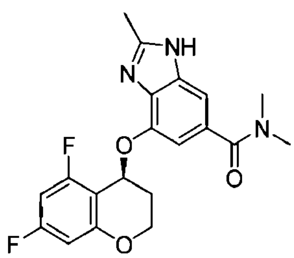

**2.** The salt of claim 1, wherein the hydrochloride is a salt represented by Formula 2 below:

[Formula 2]

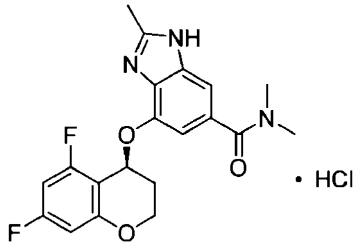

3. The salt of claim 1, wherein the hydrochloride hydrate is a salt represented by Formula 3 below:

[Formula 3]

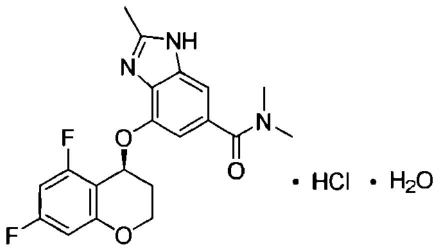

4. The salt of claim 2, wherein the salt is has an amorphous form.

5. The salt of claim 3, wherein the salt has a crystalline form.

6. The salt of claim 5, wherein the salt is a crystalline form A having a powder X-ray diffraction (PXRD) pattern comprising peaks at diffraction angles 2θ of 18.4±0.2°, 22.5±0.2°, and 25.6±0.2°.

7. The salt of claim 6, wherein the crystalline form A has a PXRD pattern comprising peaks at diffraction angles 2θ of 13.4 ±0.2°, 15.2±0.2°, 18.4±0.2°, 18.9±0.2°, 22.5±0.2°, 23.7±0.2°, 25.6±0.2°, 26.2±0.2°, and 27.7±0.2°.

8. The salt of claim 7, wherein the crystalline form A has a PXRD pattern comprising peaks at diffraction angles 2θ of 8.7 ±0.2°, 13.4±0.2°, 15.2±0.2°, 18.1±0.2°, 18.4±0.2°, 18.9±0.2°, 19.9±0.2°, 20.3±0.2°, 21.4±0.2°, 22.5±0.2°, 23.7 ±0.2°, 25.2±0.2°, 25.6±0.2°, 26.2±0.2°, 26.6±0.2°, 27.7±0.2°, 28.3±0.2°, 28.8±0.2°, and 37.5±0.2°.

9. The salt of claim 8, wherein the salt has a PXRD pattern substantially the same as that shown in FIG. 2A.

10. A pharmaceutical composition, comprising: a salt according to any one of claims 1 to 9; and a pharmaceutically acceptable carrier.

11. The pharmaceutical composition of claim 10, wherein the composition is for prevention or treatment of a disease mediated by acid pump inhibitory activity.

12. The pharmaceutical composition of claim 11, wherein the disease mediated by acid pump inhibitory activity is selected from the group consisting of gastroesophageal diseases, gastroesophageal reflux disease (GERD), peptic ulcer, gastric ulcer, duodenal ulcer, ulcer induced by NSAIDs, gastritis, Helicobacter pylori infection, dyspepsia, functional dyspepsia, Zollinger-Ellison syndrome, non-erosive reflux disease (NERD), visceral referred pain, heartburn, nausea, esophagitis, dysphagia, hypersalivation, airway disorder, and asthma.

13. The pharmaceutical composition of claim 10, wherein the pharmaceutical composition is formulated into a formulation selected from the group consisting of powder, granules, tablets, orally disintegrating tablets, capsules, a suspension, an emulsion, a syrup, aerosol, ointment, cream, suppositories, and an injectable formulation.

14. The pharmaceutical composition of claim 13, wherein the pharmaceutical composition is a tablet or orally disintegrating tablet.

FIG. 1A

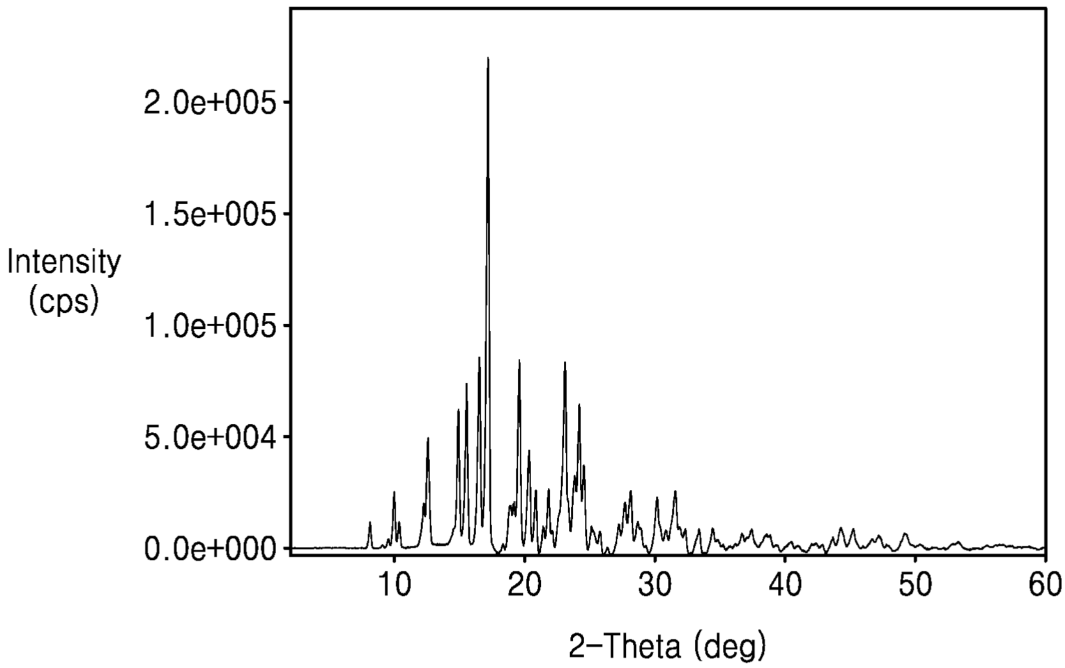
2.0e+005
1.5e+005
1.0e+005
5.0e+004
0.0e+000
Intensity
(cps)
10
20
30
40
50
60
2-Theta (deg)

FIG. 1B

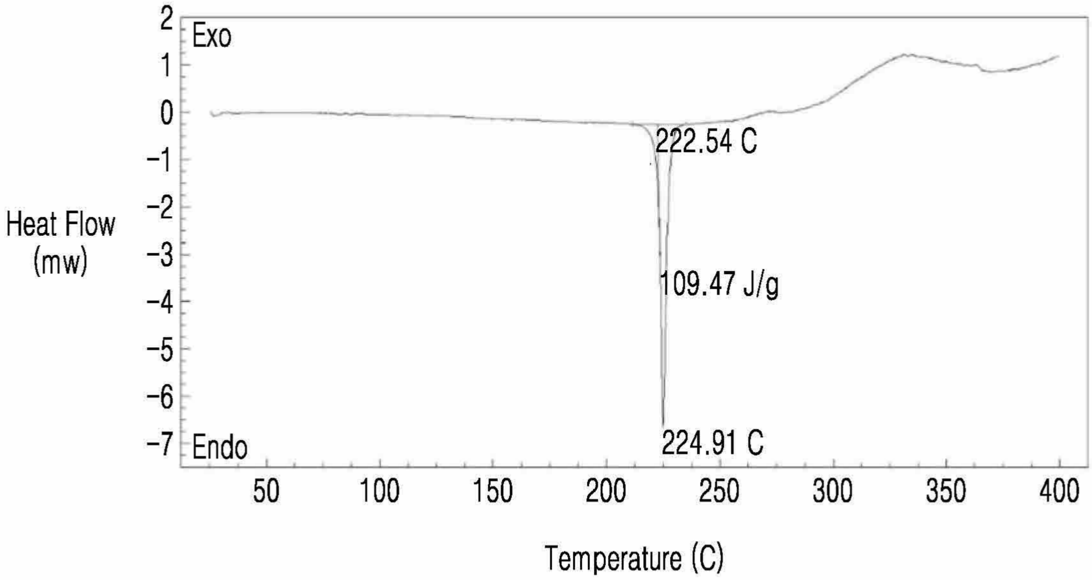
Exo
Endo
222.54 C
109.47 J/g
224.91 C
Heat Flow (mw)
2
1
0
−1
−2
−3
−4
−5
−6
−7
50
100
150
200
250
300
350
400
Temperature (C)

FIG. 2A

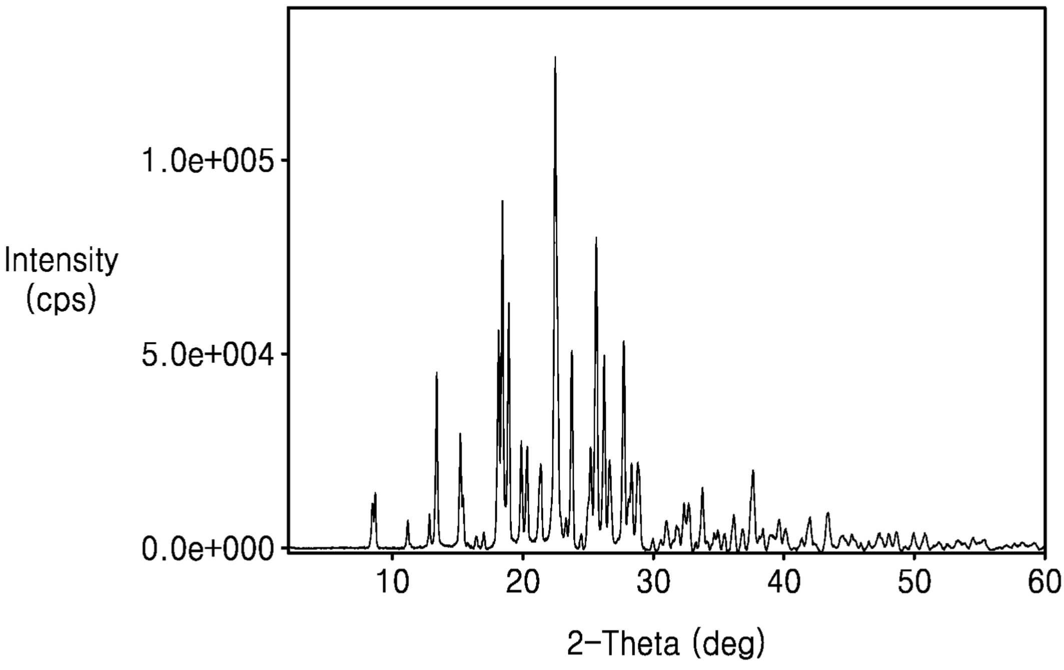
1.0e+005
5.0e+004
0.0e+000
Intensity
(cps)
10
20
30
40
50
60
2-Theta (deg)

FIG. 2B

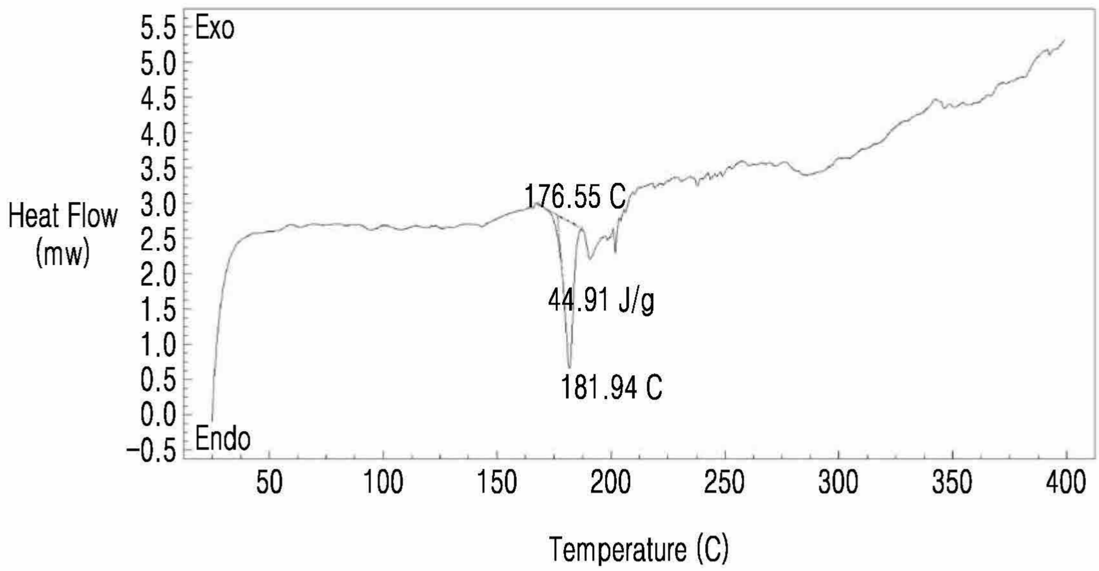
5.5
5.0
4.5
4.0
3.5
3.0
2.5
2.0
1.5
1.0
0.5
0.0
−0.5
Exo
Endo
Heat Flow
(mw)
176.55 C
44.91 J/g
181.94 C
50
100
150
200
250
300
350
400
Temperature (C)

FIG. 3A

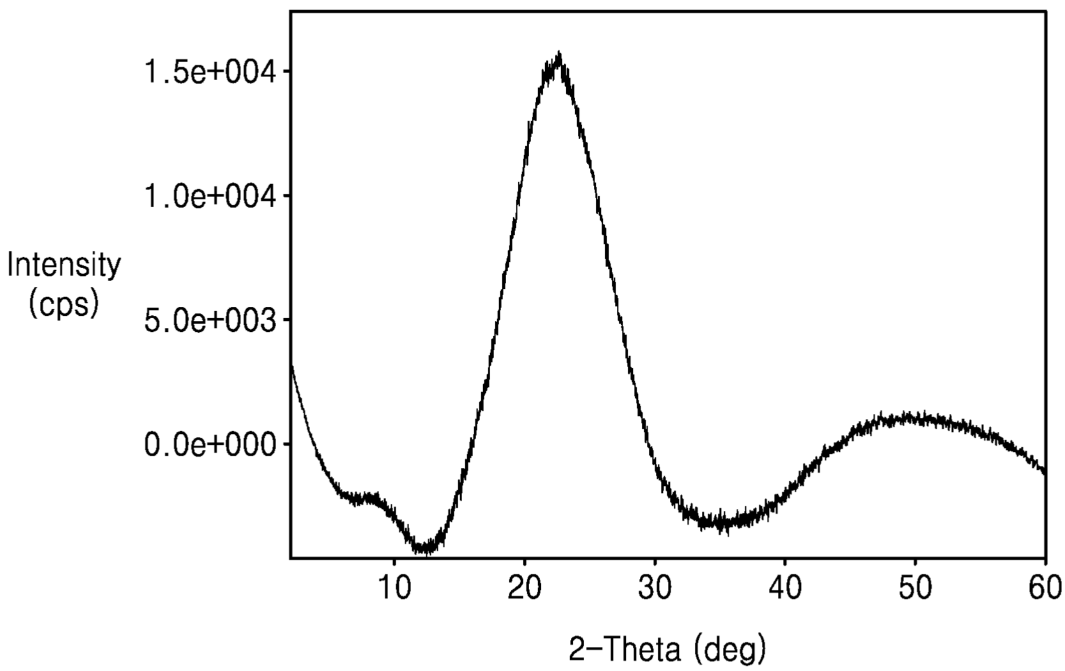
1.5e+004
1.0e+004
Intensity
(cps)
5.0e+003
0.0e+000
10
20
30
40
50
60
2–Theta (deg)

FIG. 3B

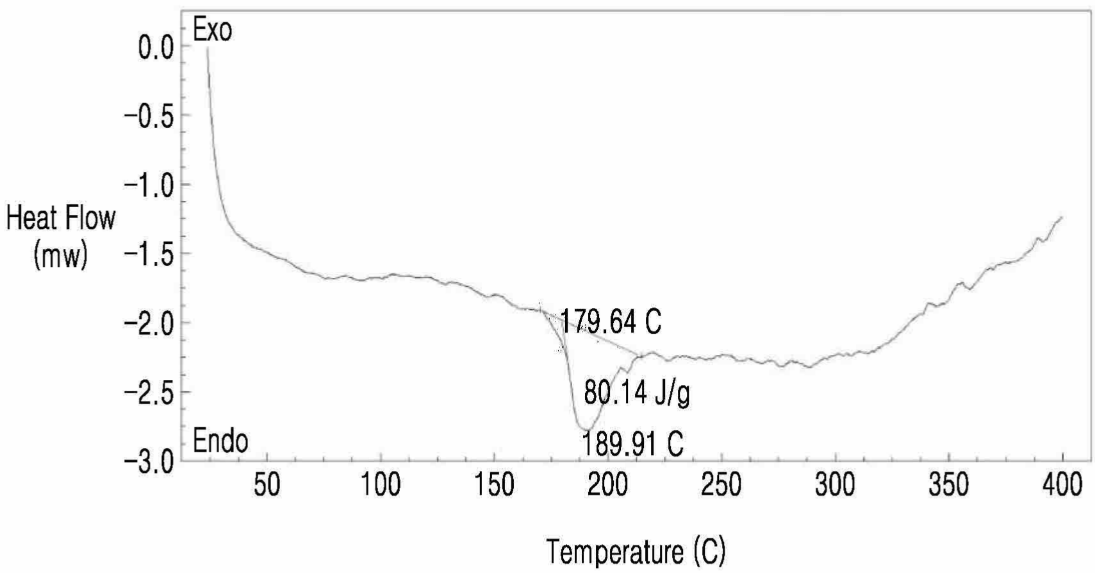
Exo
Endo
Heat Flow
(mw)
0.0
-0.5
-1.0
-1.5
-2.0
-2.5
-3.0
179.64 C
80.14 J/g
189.91 C
50
100
150
200
250
300
350
400
Temperature (C)

FIG. 4A

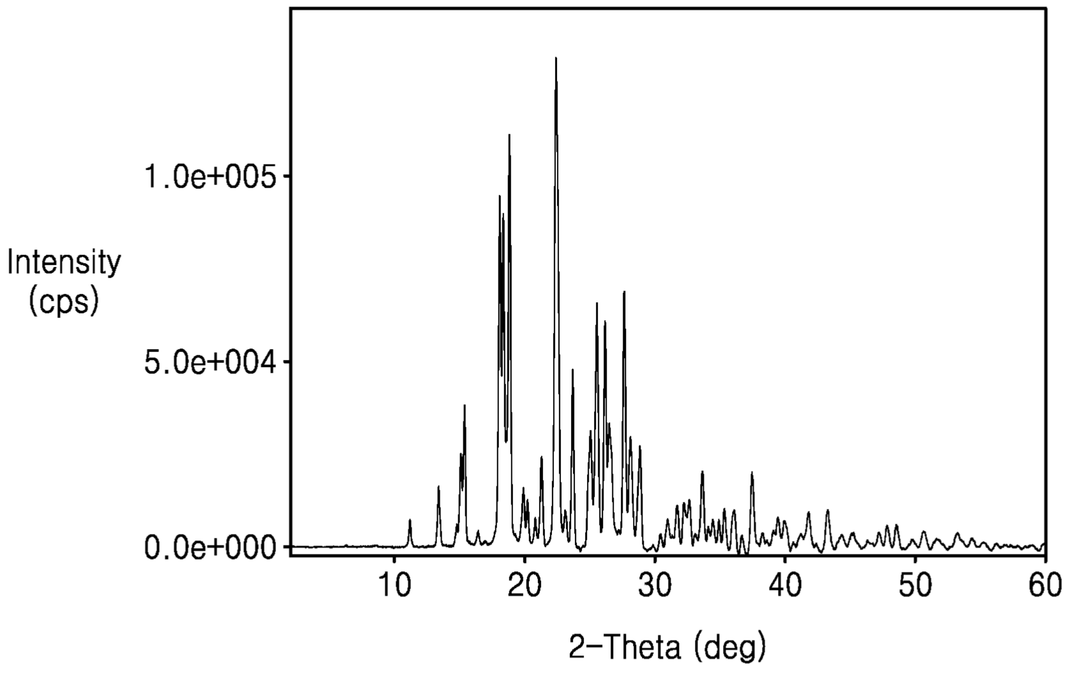
1.0e+005
5.0e+004
0.0e+000
Intensity
(cps)
10
20
30
40
50
60
2-Theta (deg)

FIG. 4B

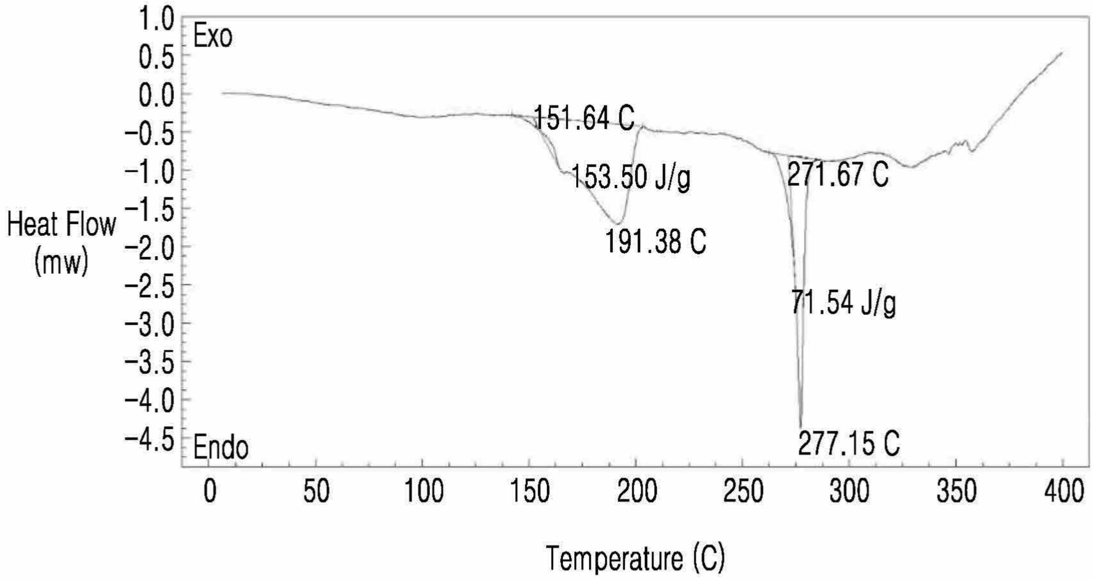
Exo
Endo
151.64 C
153.50 J/g
191.38 C
271.67 C
71.54 J/g
277.15 C
Heat Flow (mw)
Temperature (C)
1.0
0.5
0.0
-0.5
-1.0
-1.5
-2.0
-2.5
-3.0
-3.5
-4.0
-4.5
0
50
100
150
200
250
300
350
400

FIG. 5A

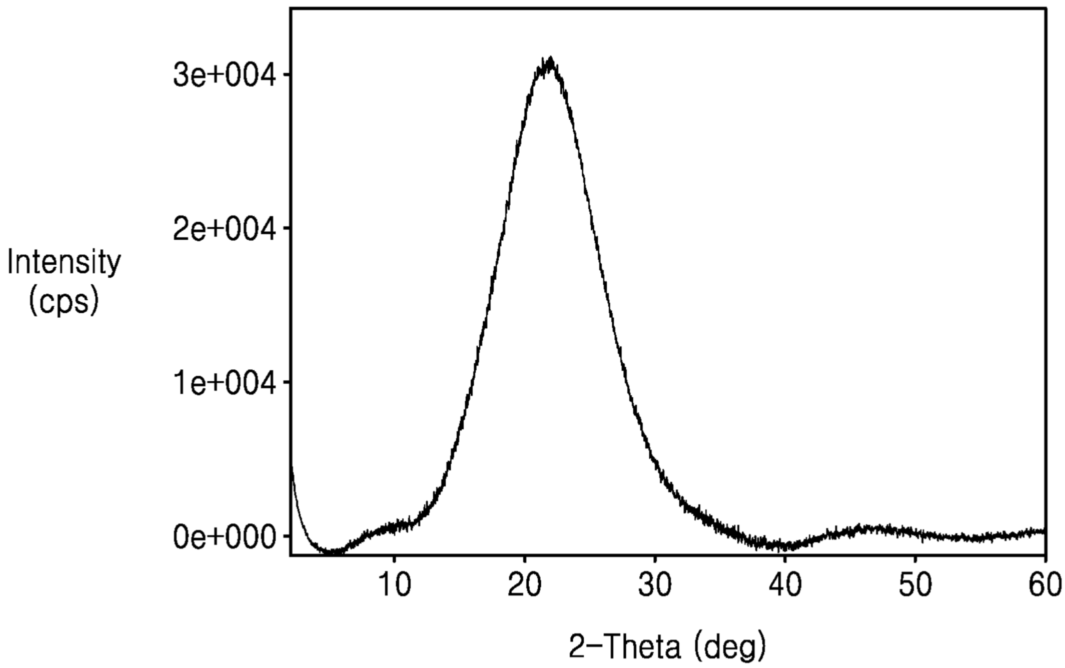
3e+004
2e+004
1e+004
0e+000
Intensity
(cps)
10
20
30
40
50
60
2-Theta (deg)

FIG. 5B

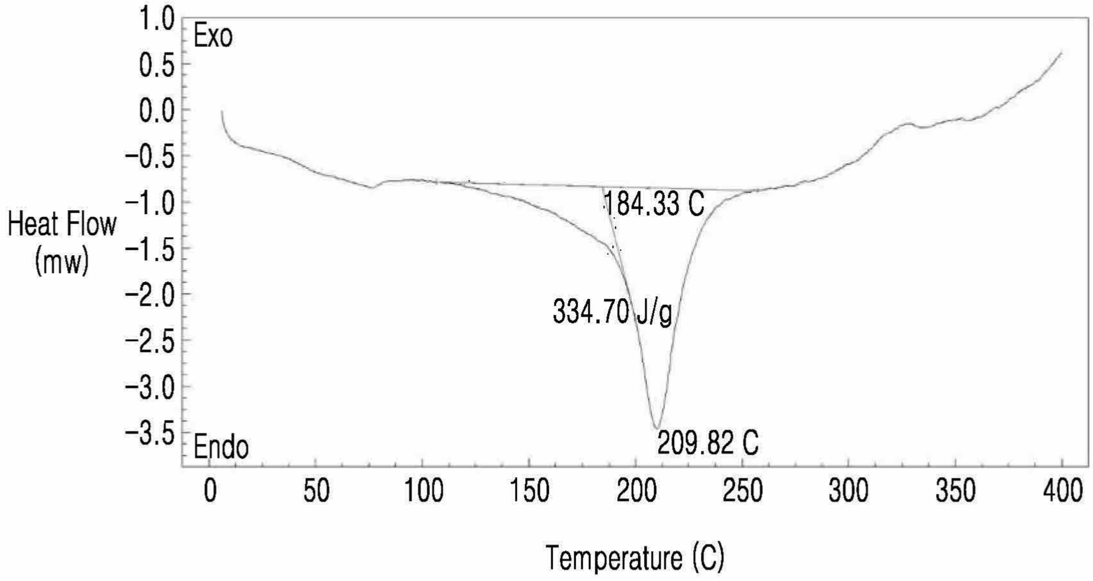
1.0
0.5
0.0
−0.5
−1.0
−1.5
−2.0
−2.5
−3.0
−3.5
Exo
Endo
Heat Flow (mw)
184.33 C
334.70 J/g
209.82 C
0
50
100
150
200
250
300
350
400
Temperature (C)

FIG. 6A

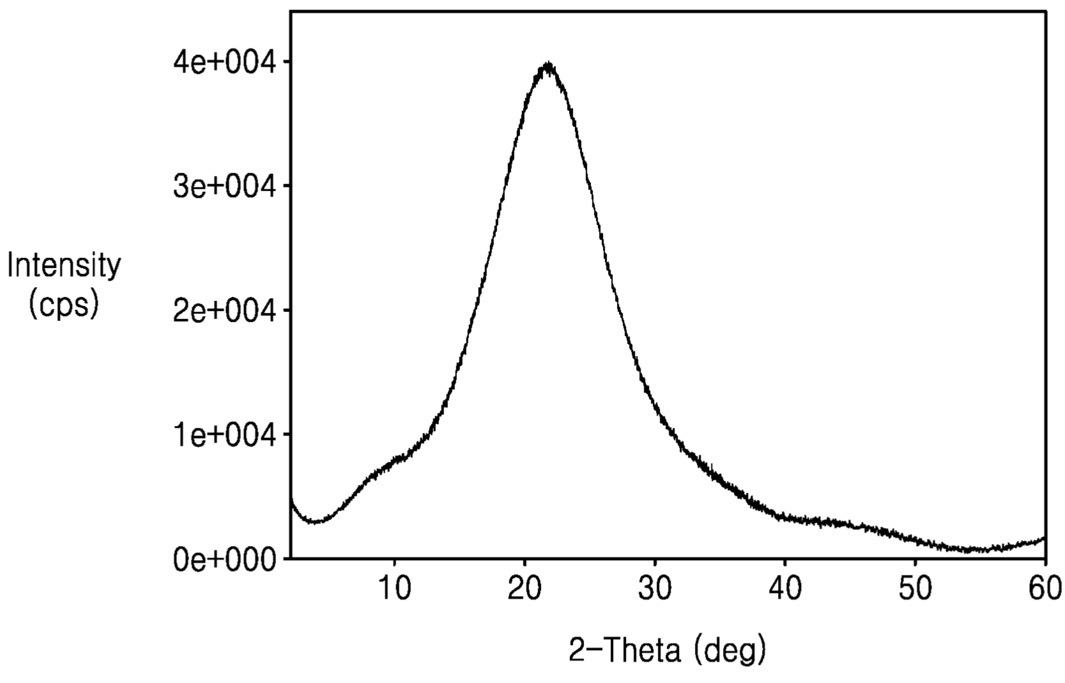

4e+004
3e+004
Intensity
(cps)
2e+004
1e+004
0e+000
10
20
30
40
50
60
2-Theta (deg)

FIG. 6B

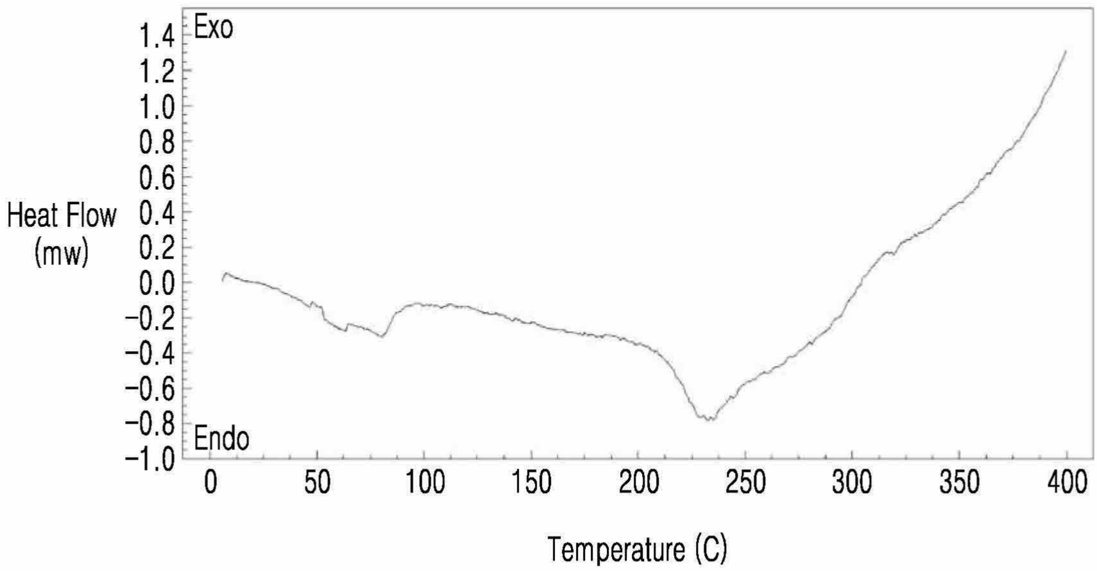
Exo
Endo
Heat Flow (mw)
1.4
1.2
1.0
0.8
0.6
0.4
0.2
0.0
−0.2
−0.4
−0.6
−0.8
−1.0
0
50
100
150
200
250
300
350
400
Temperature (C)

FIG. 7A

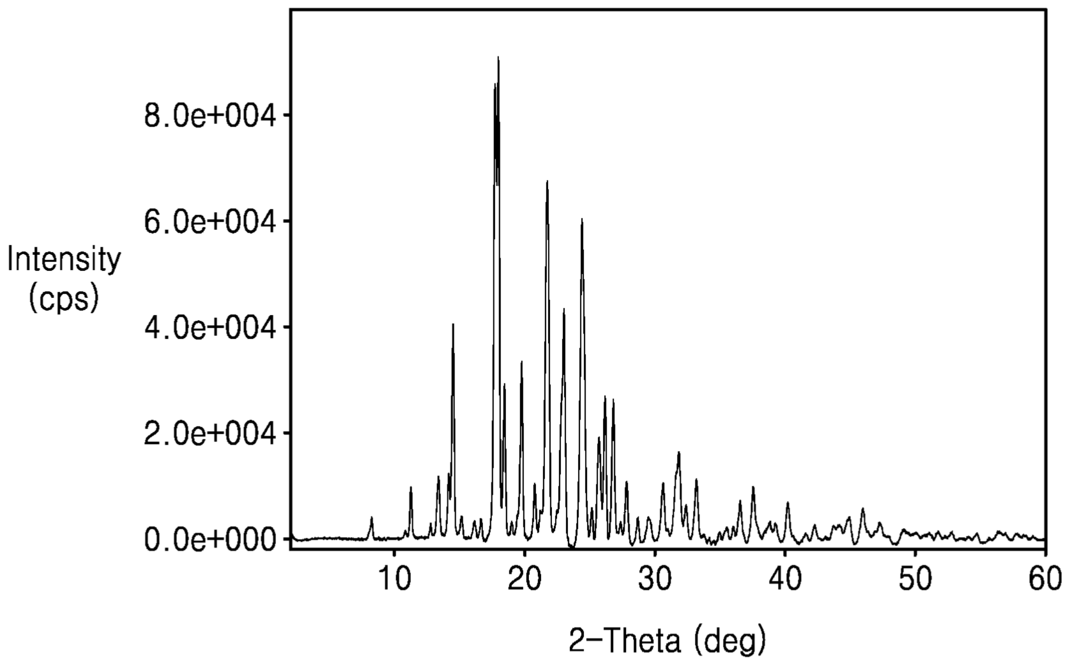
8.0e+004
6.0e+004
4.0e+004
2.0e+004
0.0e+000
Intensity
(cps)
10
20
30
40
50
60
2-Theta (deg)

FIG. 7B

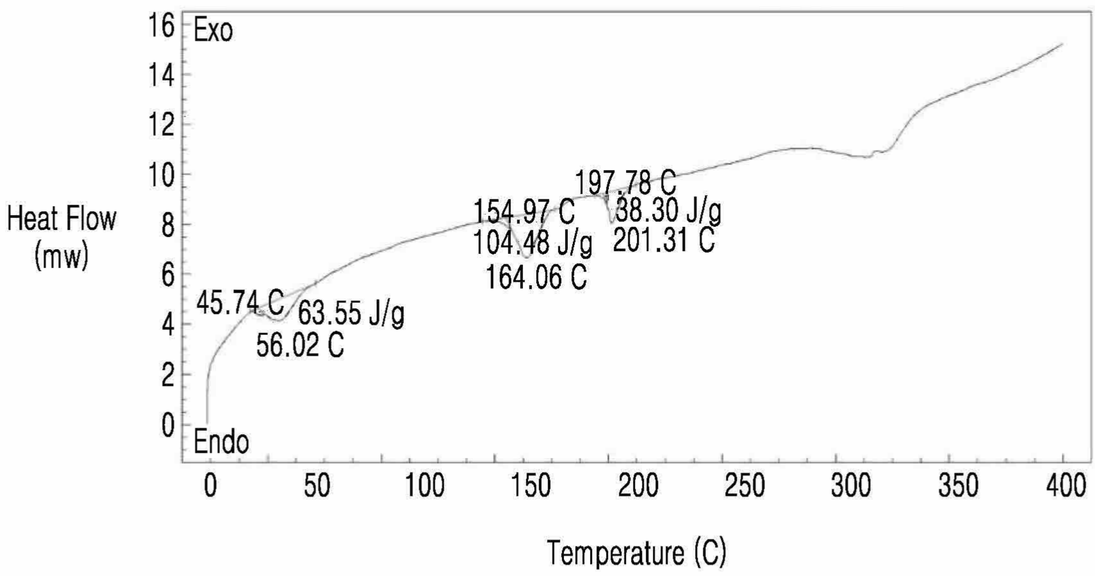
16
14
12
10
8
6
4
2
0
Exo
Endo
Heat Flow
(mw)
45.74 C
63.55 J/g
56.02 C
154.97 C
104.48 J/g
164.06 C
197.78 C
38.30 J/g
201.31 C
0
50
100
150
200
250
300
350
400
Temperature (C)

FIG. 7C

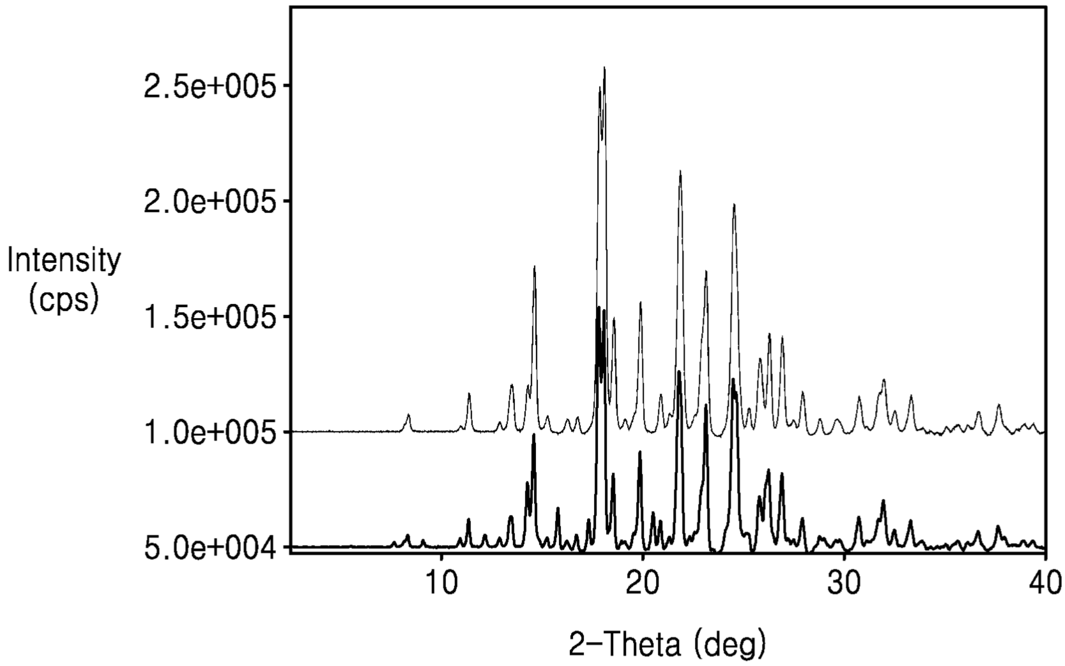
2.5e+005
2.0e+005
1.5e+005
1.0e+005
5.0e+004
Intensity (cps)
10
20
30
40
2-Theta (deg)

FIG. 8A

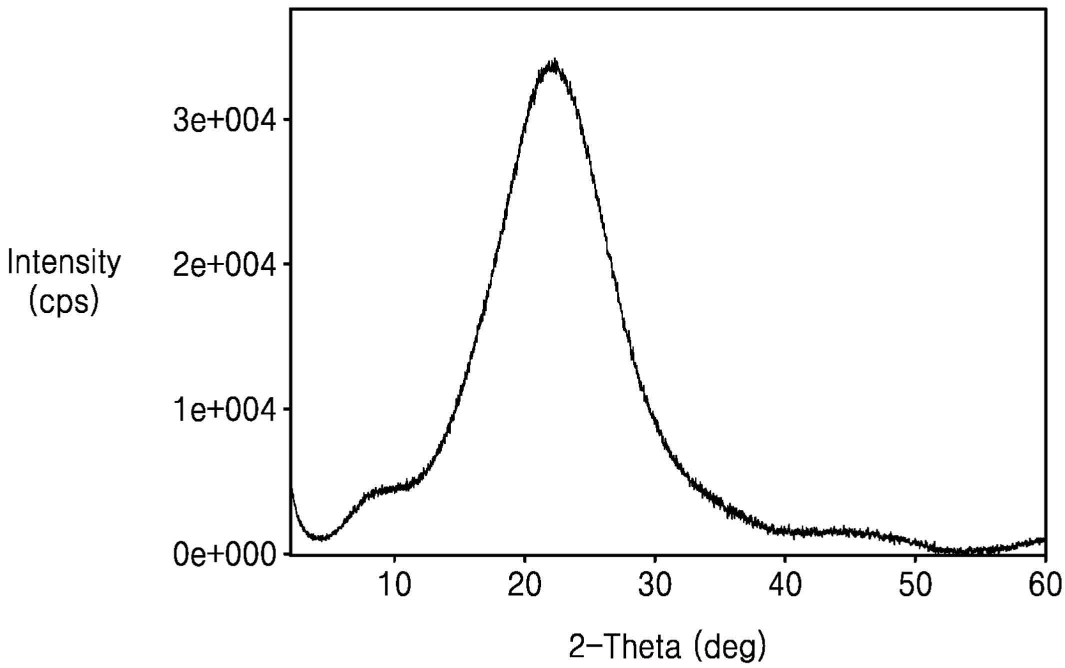
Intensity
(cps)
3e+004
2e+004
1e+004
0e+000
10
20
30
40
50
60
2-Theta (deg)

FIG. 8B

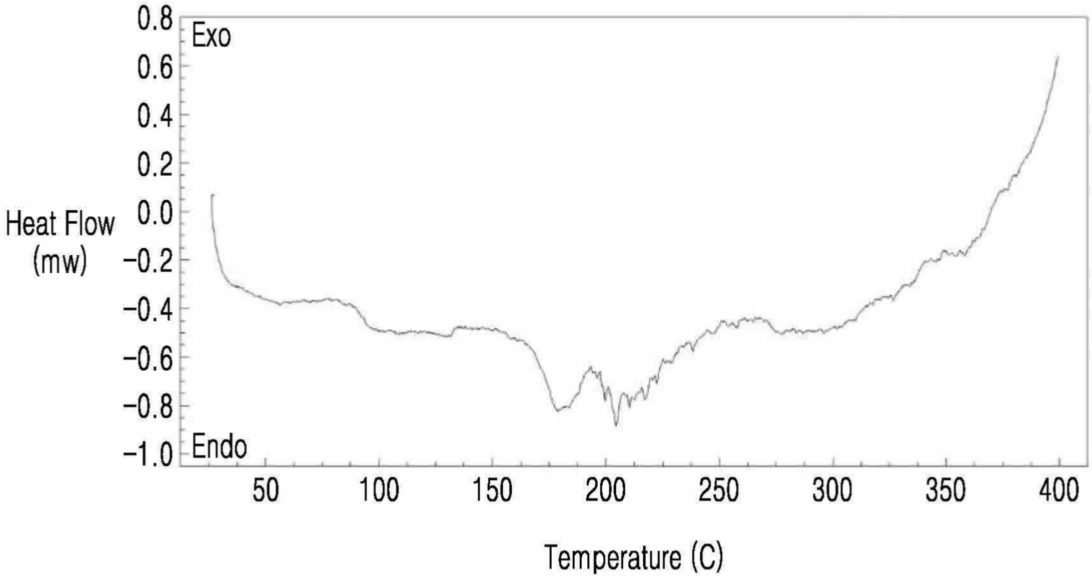
0.8
0.6
0.4
0.2
0.0
-0.2
-0.4
-0.6
-0.8
-1.0
Exo
Endo
Heat Flow
(mw)
50
100
150
200
250
300
350
400
Temperature (C)

FIG. 9A

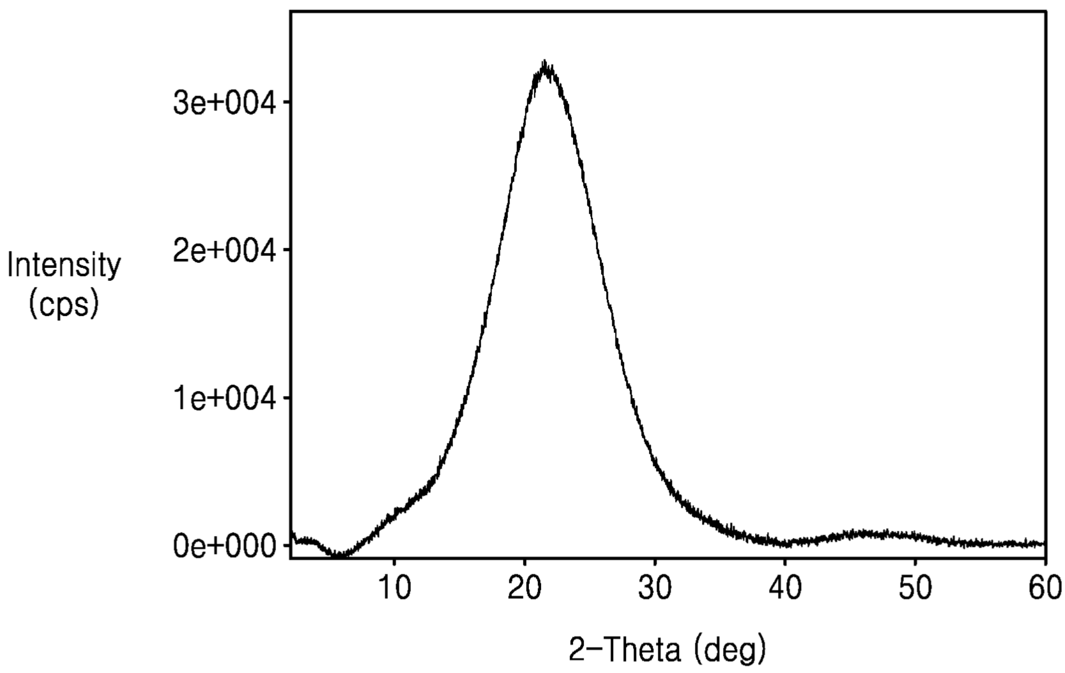
Intensity (cps)
3e+004
2e+004
1e+004
0e+000
10
20
30
40
50
60
2-Theta (deg)

FIG. 9B

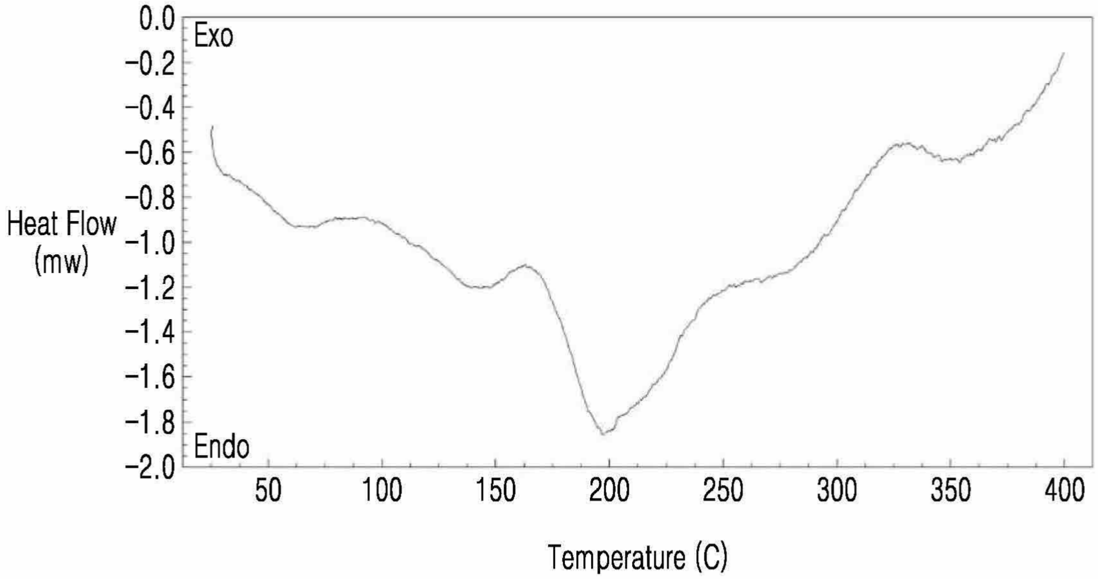
0.0
−0.2
−0.4
−0.6
−0.8
−1.0
−1.2
−1.4
−1.6
−1.8
−2.0
Exo
Endo
Heat Flow (mw)
50
100
150
200
250
300
350
400
Temperature (C)

FIG. 10A

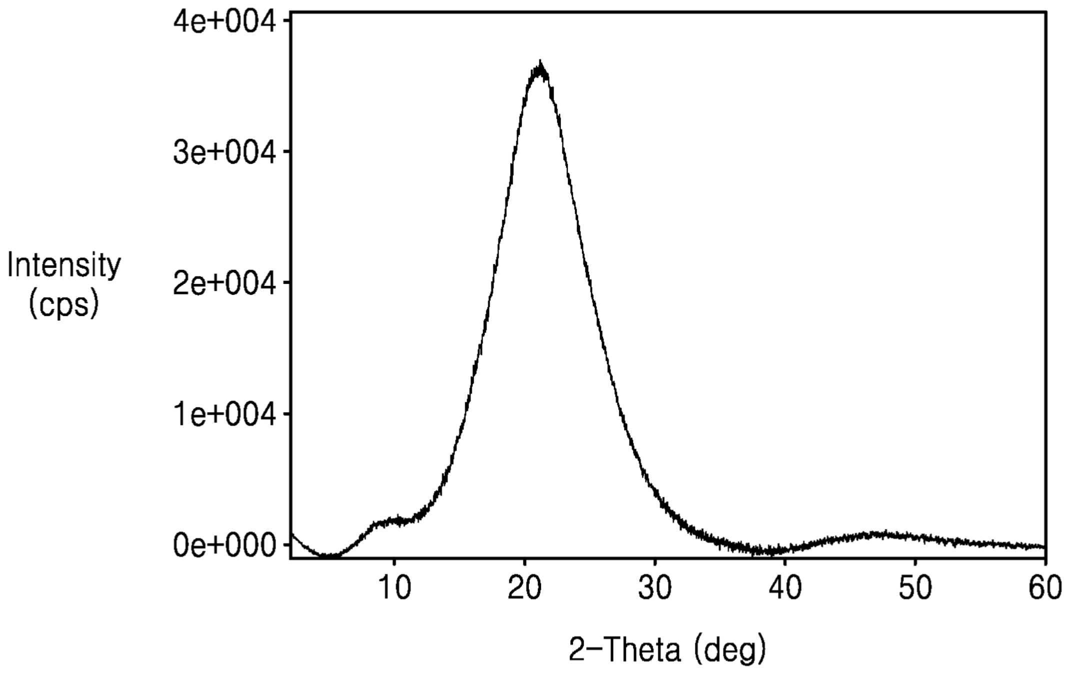
4e+004
3e+004
2e+004
1e+004
0e+000
Intensity
(cps)
10
20
30
40
50
60
2-Theta (deg)

FIG. 10B

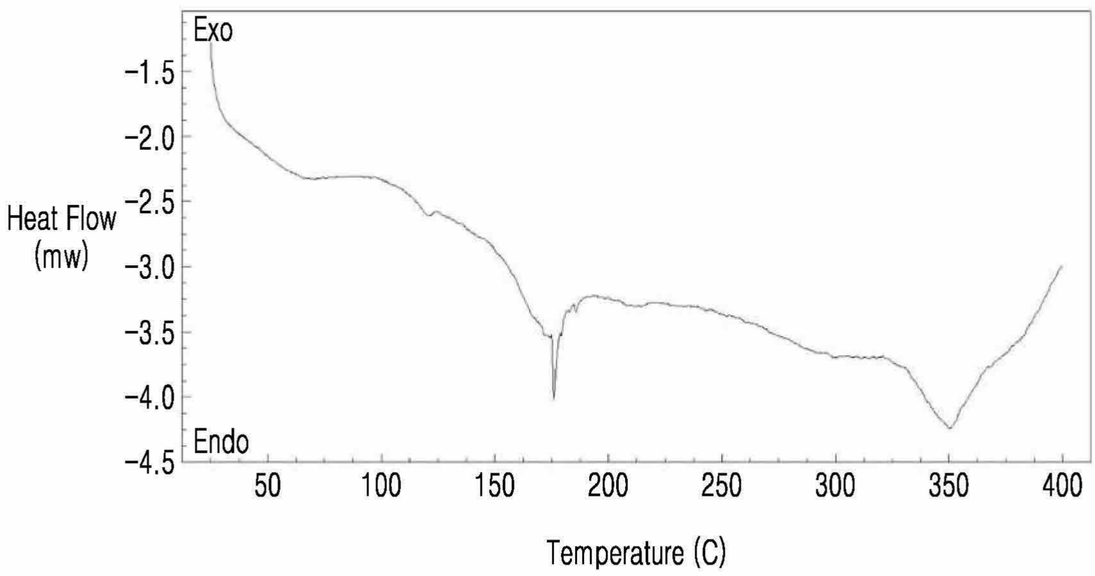
Exo
Endo
Heat Flow (mw)
-1.5
-2.0
-2.5
-3.0
-3.5
-4.0
-4.5
50
100
150
200
250
300
350
400
Temperature (C)

FIG. 11A

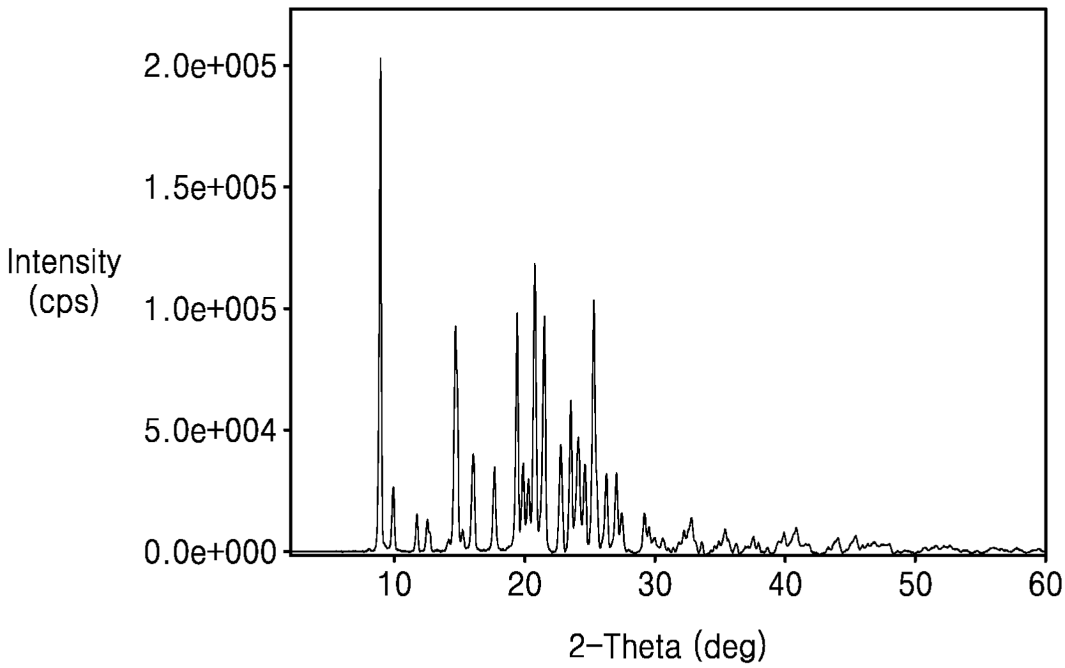
2.0e+005
1.5e+005
1.0e+005
5.0e+004
0.0e+000
Intensity
(cps)
10
20
30
40
50
60
2-Theta (deg)

FIG. 11B

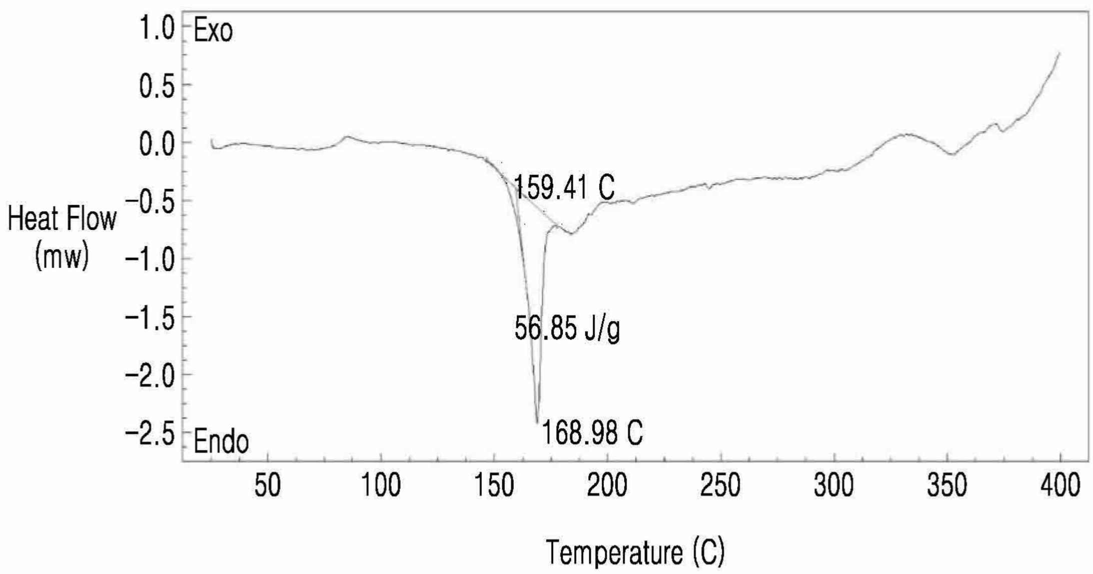
1.0
0.5
0.0
-0.5
-1.0
-1.5
-2.0
-2.5
Exo
Endo
Heat Flow
(mw)
159.41 C
56.85 J/g
168.98 C
50
100
150
200
250
300
350
400
Temperature (C)

FIG. 12A

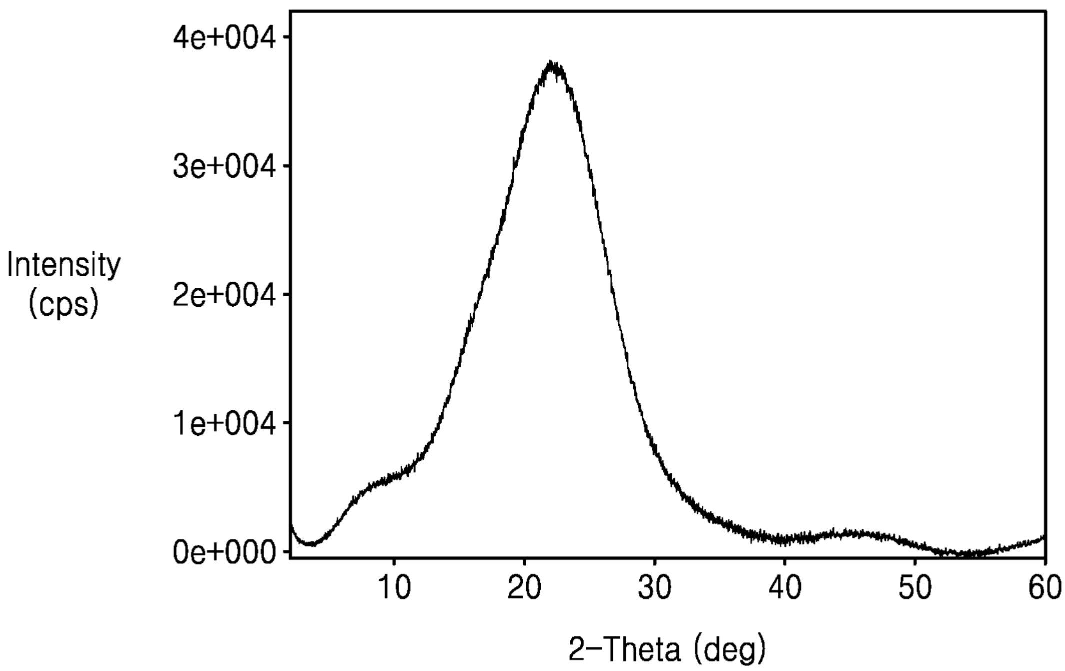
Intensity
(cps)
4e+004
3e+004
2e+004
1e+004
0e+000
10
20
30
40
50
60
2-Theta (deg)

FIG. 12B

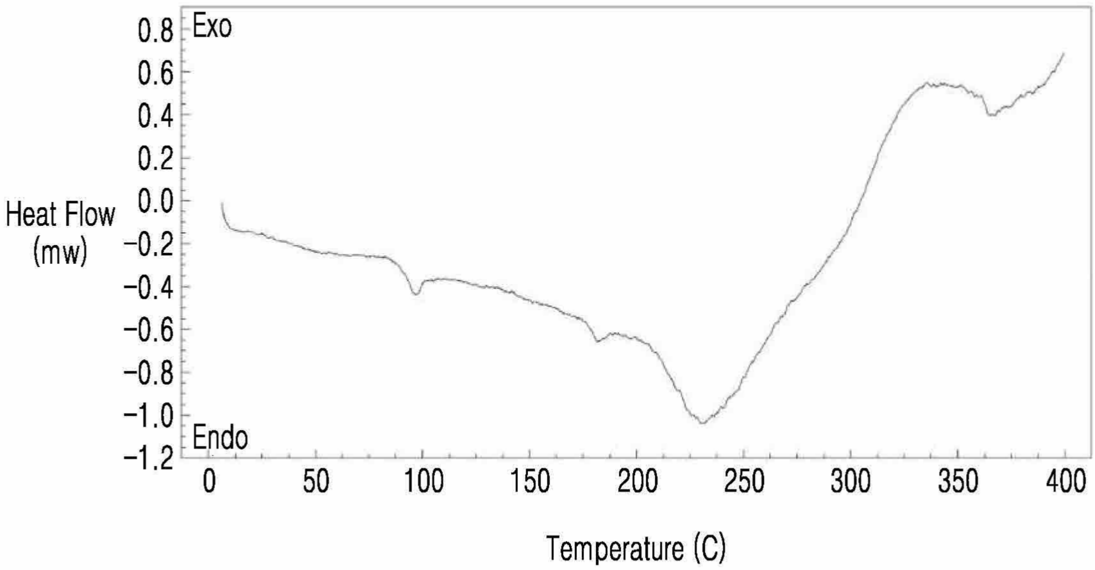
Exo
Endo
Heat Flow
(mw)
0.8
0.6
0.4
0.2
0.0
−0.2
−0.4
−0.6
−0.8
−1.0
−1.2
0
50
100
150
200
250
300
350
400
Temperature (C)

FIG. 13

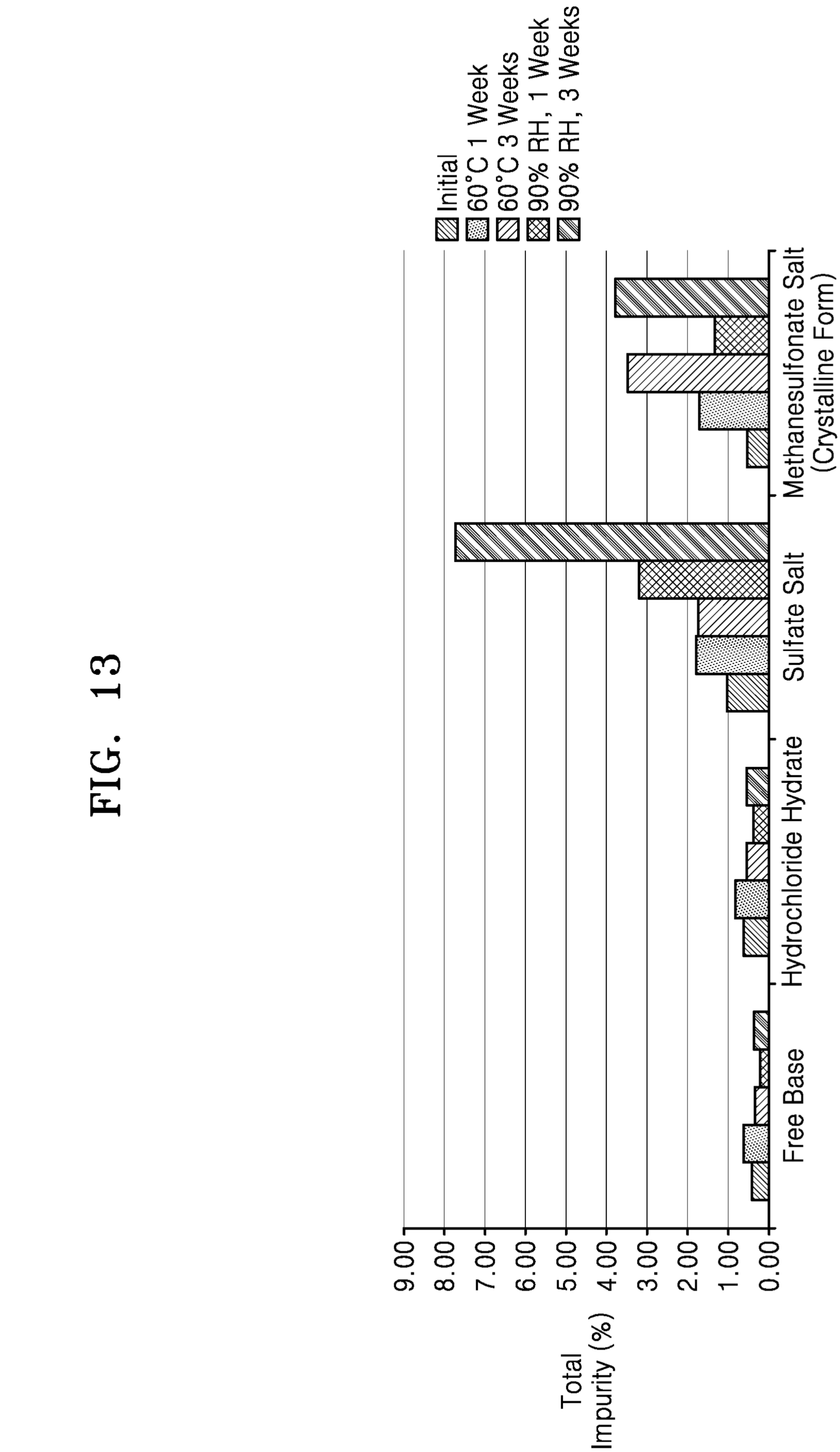
Total Impurity (%)
9.00
8.00
7.00
6.00
5.00
4.00
3.00
2.00
1.00
0.00
Free Base
Hydrochloride Hydrate
Sulfate Salt
Methanesulfonate Salt (Crystalline Form)
Initial
60°C 1 Week
60°C 3 Weeks
90% RH, 1 Week
90% RH, 3 Weeks

FIG. 14

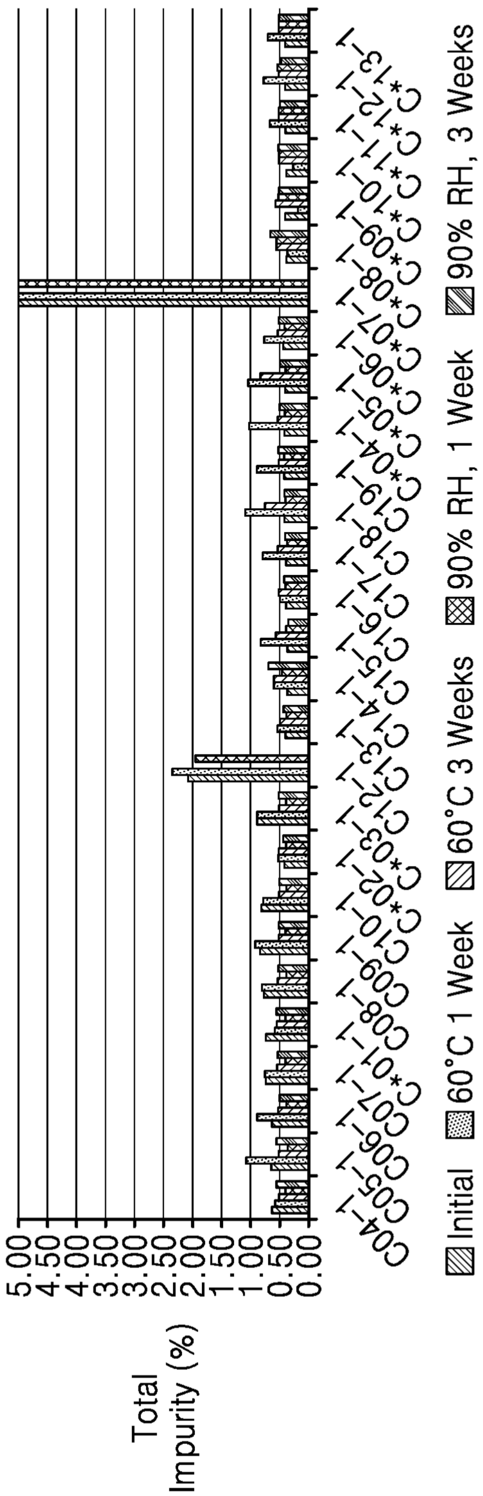
Total Impurity (%)
5.00
4.50
4.00
3.50
3.00
2.50
2.00
1.50
1.00
0.50
0.00
C04-1
C05-1
C06-1
C07-1
C*01-1
C08-1
C09-1
C10-1
C*02-1
C*03-1
C12-1
C13-1
C14-1
C15-1
C16-1
C17-1
C18-1
C19-1
C*04-1
C*05-1
C*06-1
C*07-1
C*08-1
C*09-1
C*10-1
C*11-1
C*12-1
C*13-1
Initial
60°C 1 Week
60°C 3 Weeks
90% RH, 1 Week
90% RH, 3 Weeks

FIG. 15

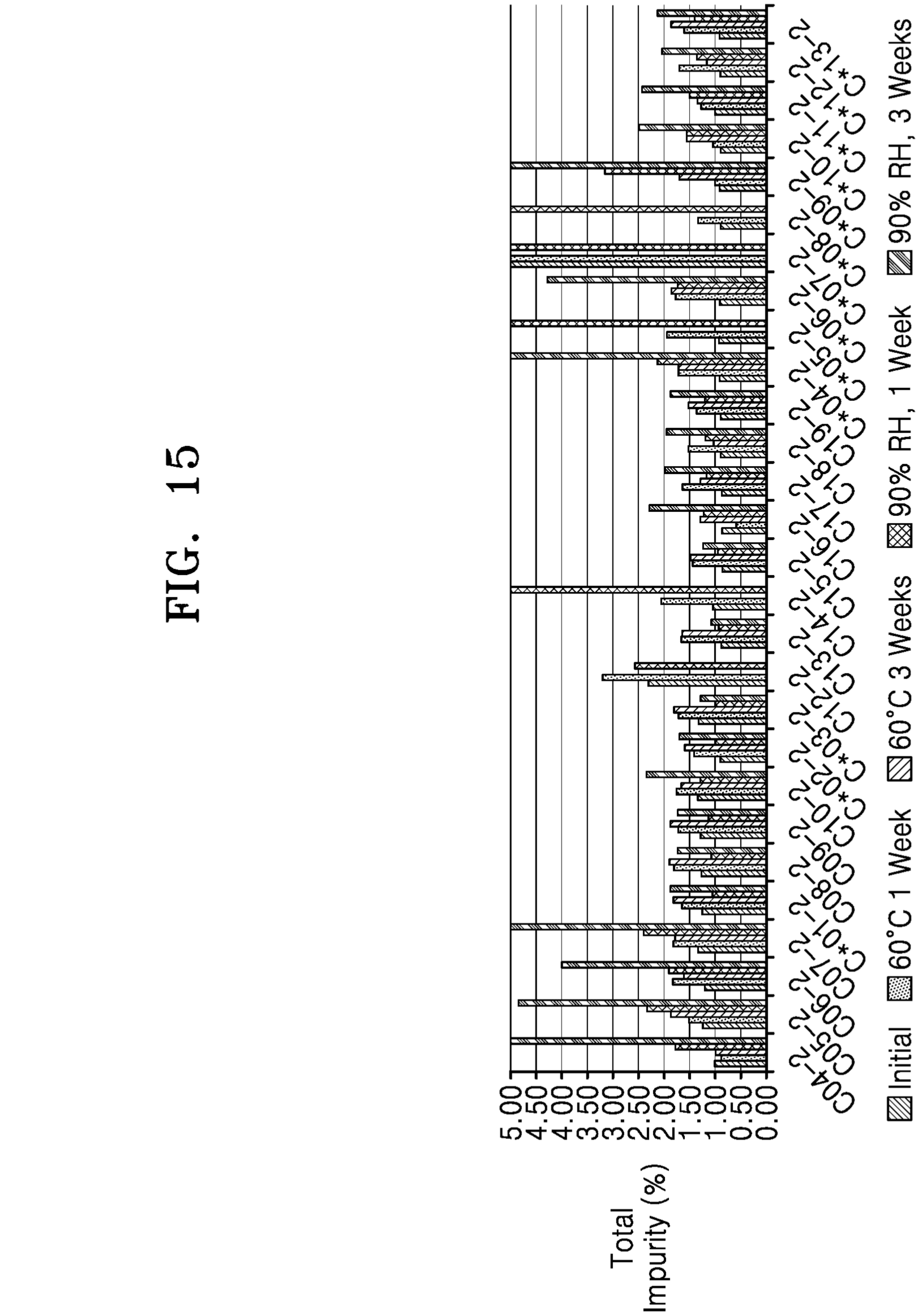
Total Impurity (%)
0.00
0.50
1.00
1.50
2.00
2.50
3.00
3.50
4.00
4.50
5.00
C04-2
C05-2
C06-2
C07-2
C*01-2
C08-2
C09-2
C10-2
C*02-2
C*03-2
C12-2
C13-2
C14-2
C15-2
C16-2
C17-2
C18-2
C19-2
C*04-2
C*05-2
C*06-2
C*07-2
C*08-2
C*09-2
C*10-2
C*11-2
C*12-2
C*13-2
Initial
60°C 1 Week
60°C 3 Weeks
90% RH, 1 Week
90% RH, 3 Weeks

# FIG. 16

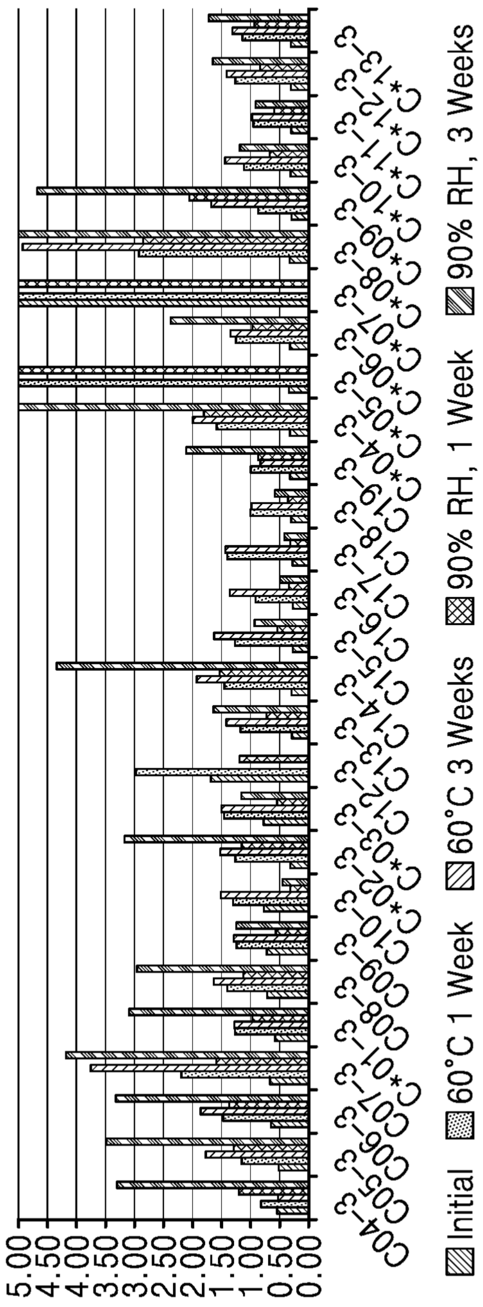
Total Impurity (%)
5.00
4.50
4.00
3.50
3.00
2.50
2.00
1.50
1.00
0.50
0.00
C04-3
C05-3
C06-3
C07-3
C*01-3
C08-3
C09-3
C10-3
C*02-3
C*03-3
C12-3
C13-3
C14-3
C15-3
C16-3
C17-3
C18-3
C19-3
C*04-3
C*05-3
C*06-3
C*07-3
C*08-3
C*09-3
C*10-3
C*11-3
C*12-3
C*13-3
Initial
60°C 1 Week
60°C 3 Weeks
90% RH, 1 Week
90% RH, 3 Weeks

FIG. 17

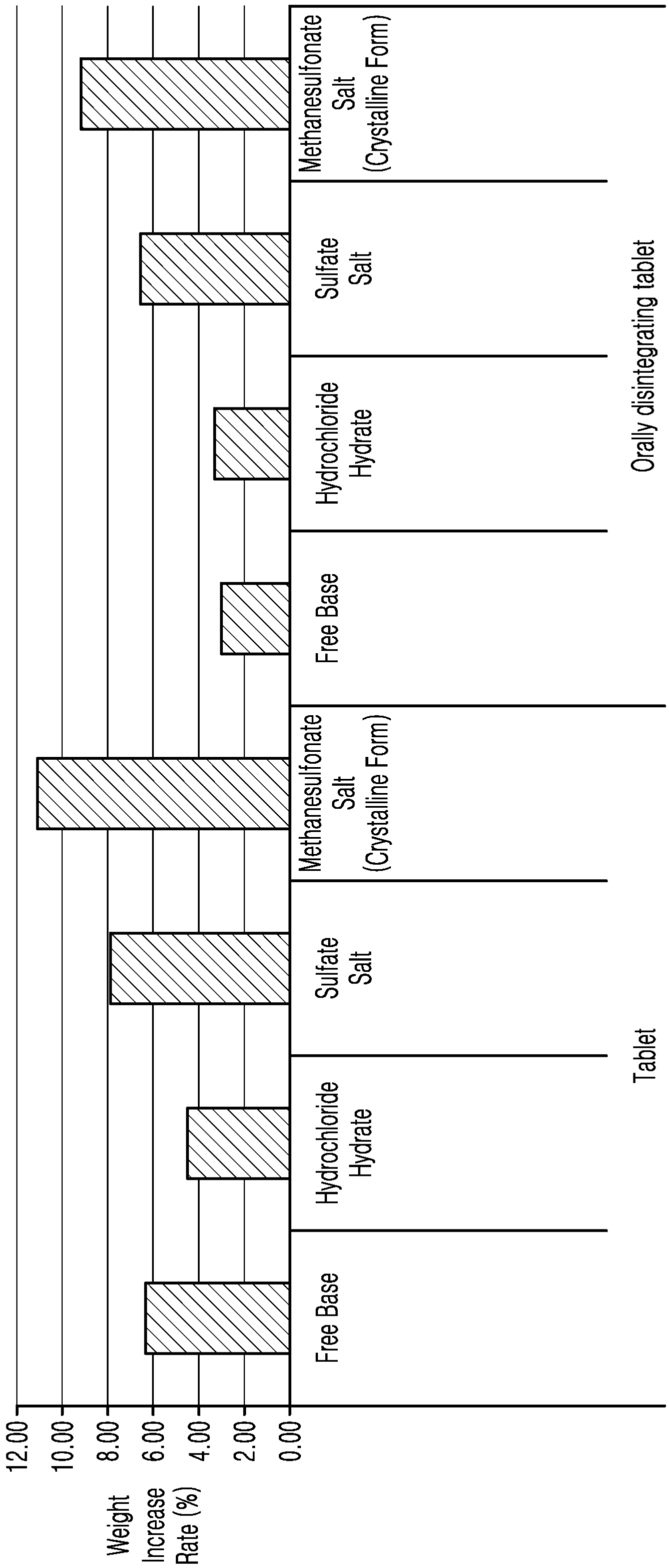
Weight Increase Rate (%)
12.00
10.00
8.00
6.00
4.00
2.00
0.00
Free Base
Hydrochloride Hydrate
Sulfate Salt
Methanesulfonate Salt (Crystalline Form)
Tablet
Free Base
Hydrochloride Hydrate
Sulfate Salt
Methanesulfonate Salt (Crystalline Form)
Orally disintegrating tablet

FIG. 18

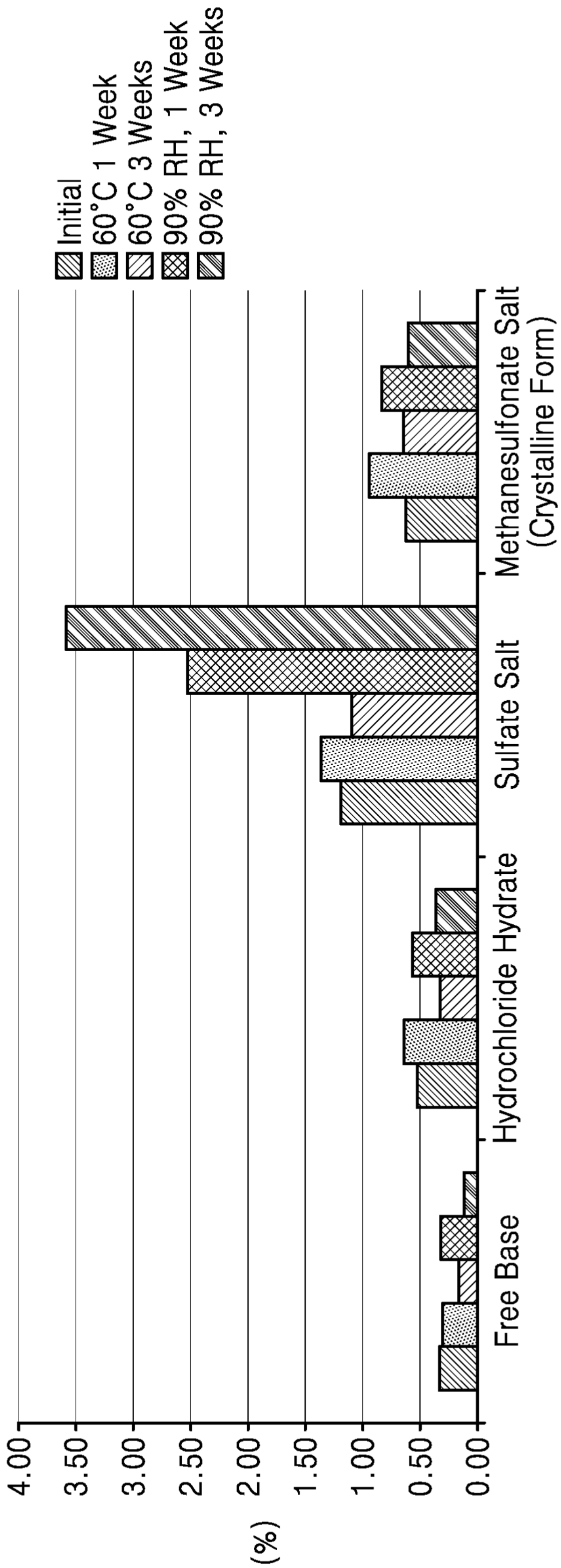
Total Impurity (%)
0.00
0.50
1.00
1.50
2.00
2.50
3.00
3.50
4.00
Free Base
Hydrochloride Hydrate
Sulfate Salt
Methanesulfonate Salt (Crystalline Form)
Initial
60°C 1 Week
60°C 3 Weeks
90% RH, 1 Week
90% RH, 3 Weeks

FIG. 19

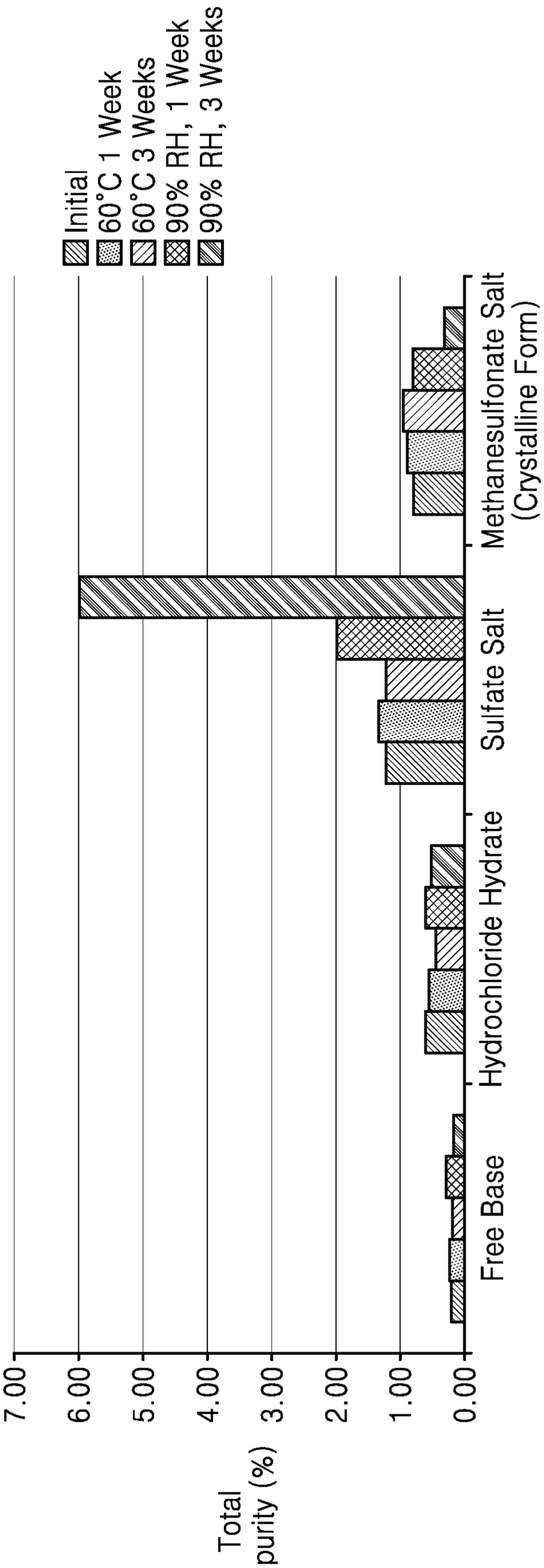
Total Impurity (%)
7.00
6.00
5.00
4.00
3.00
2.00
1.00
0.00
Free Base
Hydrochloride Hydrate
Sulfate Salt
Methanesulfonate Salt (Crystalline Form)
Initial
60°C 1 Week
60°C 3 Weeks
90% RH, 1 Week
90% RH, 3 Weeks

# FIG. 20

| Type | K-CAB Orally Disintegrating Tablet 50 mg | Test Formulation (Orally Disintegrating Tablet) | | |
|---|---|---|---|---|
| | Free Base | Hydrochloride Hydrate | Sulfate Salt | Methanesulfonate Salt (Crystalline Form) |
| Wetting Time | 33 Seconds | 22 Seconds | 38 Seconds | 55 Seconds |
| Appearance | 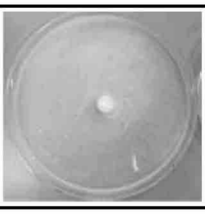 | 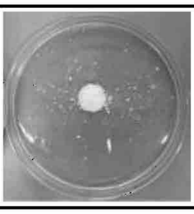 | 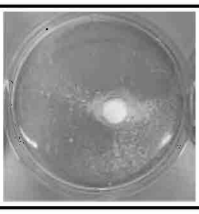 | 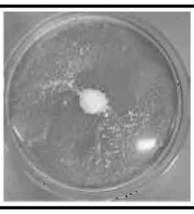 |

# FIG. 21

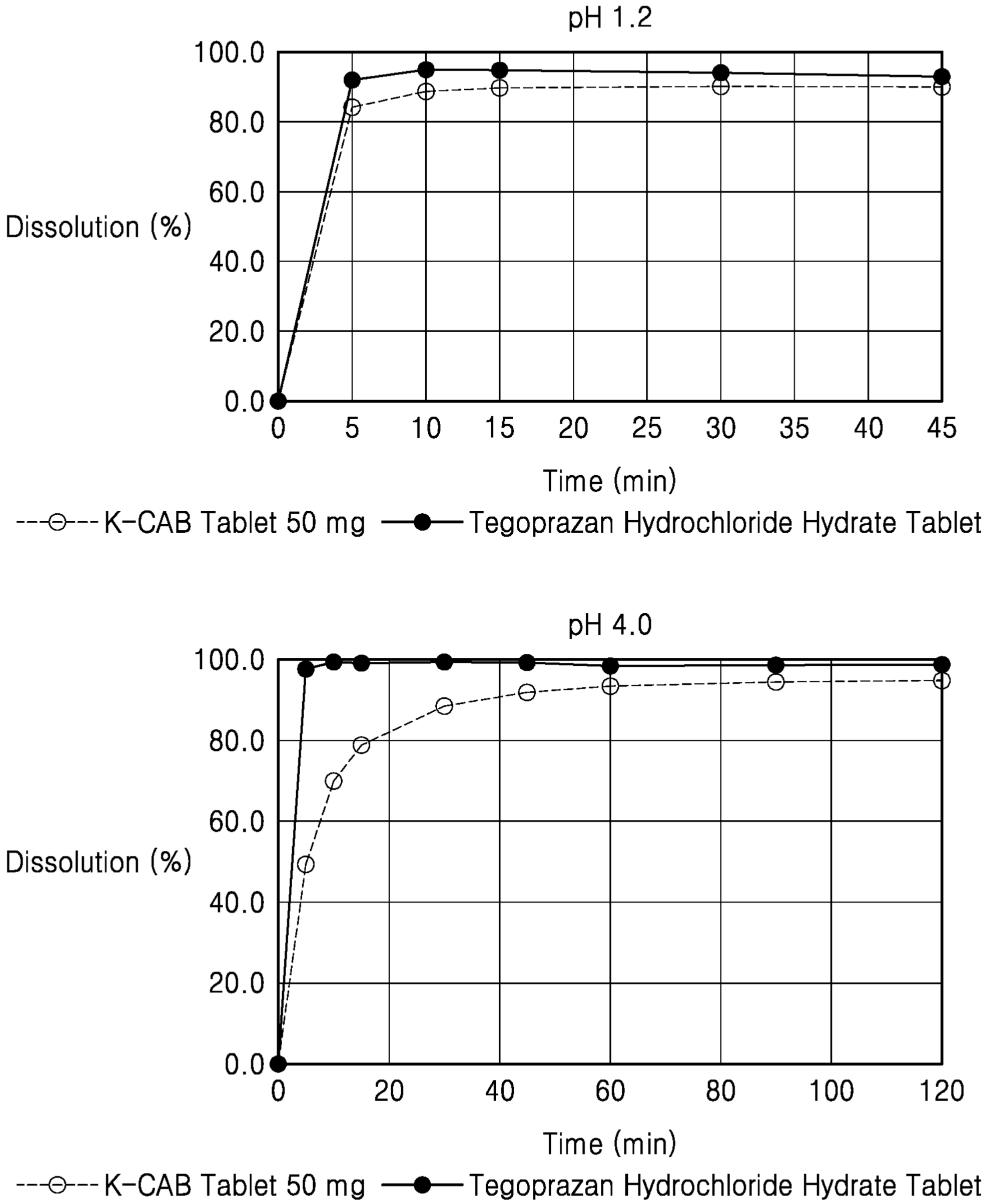
pH 1.2
100.0
80.0
60.0
40.0
20.0
0.0
Dissolution (%)
0
5
10
15
20
25
30
35
40
45
Time (min)
K-CAB Tablet 50 mg
Tegoprazan Hydrochloride Hydrate Tablet
pH 4.0
100.0
80.0
60.0
40.0
20.0
0.0
Dissolution (%)
0
20
40
60
80
100
120
Time (min)
K-CAB Tablet 50 mg
Tegoprazan Hydrochloride Hydrate Tablet

FIG. 22

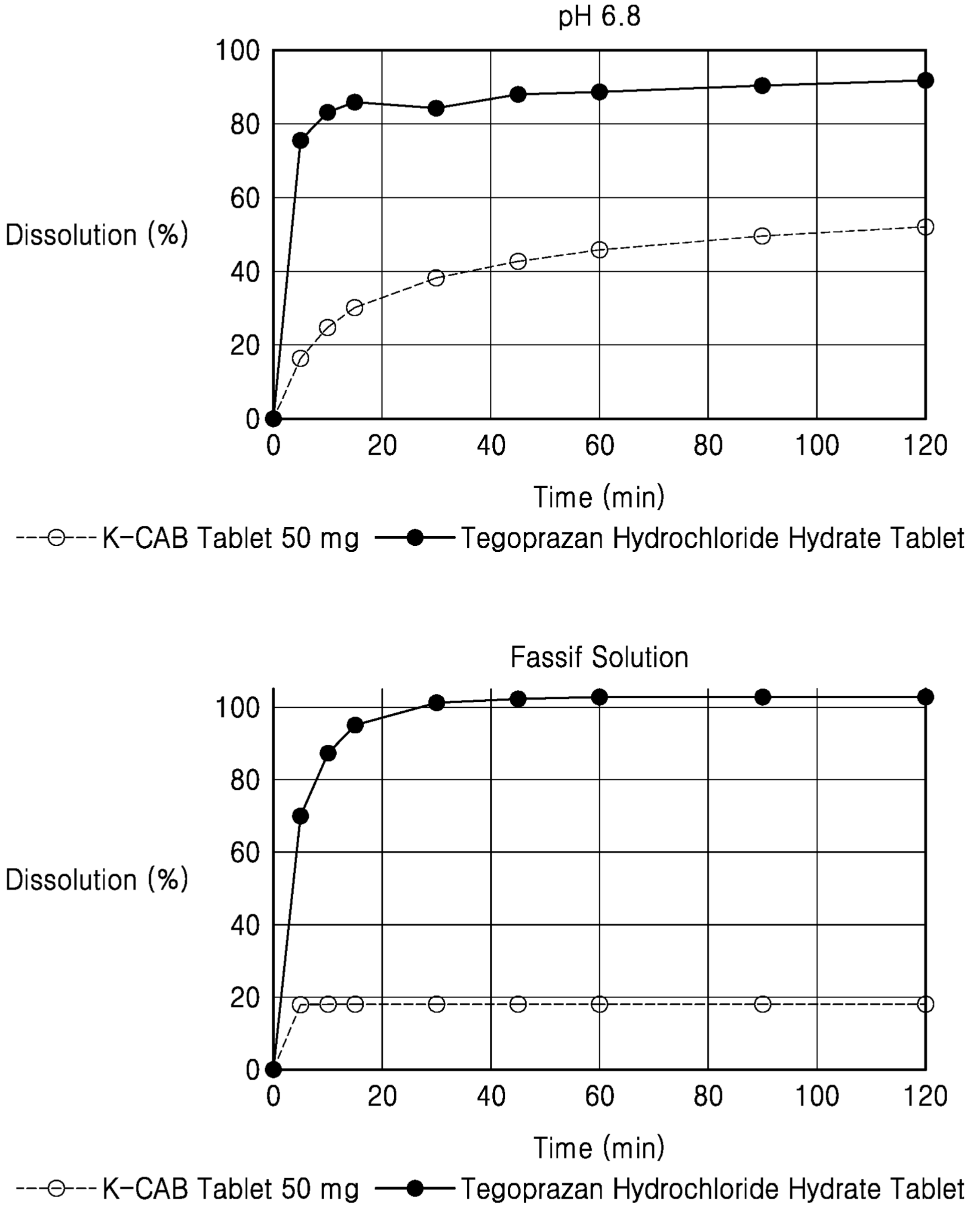
pH 6.8
100
80
60
40
20
0
Dissolution (%)
0
20
40
60
80
100
120
Time (min)
K-CAB Tablet 50 mg
Tegoprazan Hydrochloride Hydrate Tablet
Fassif Solution
100
80
60
40
20
0
Dissolution (%)
0
20
40
60
80
100
120
Time (min)
K-CAB Tablet 50 mg
Tegoprazan Hydrochloride Hydrate Tablet

FIG. 23

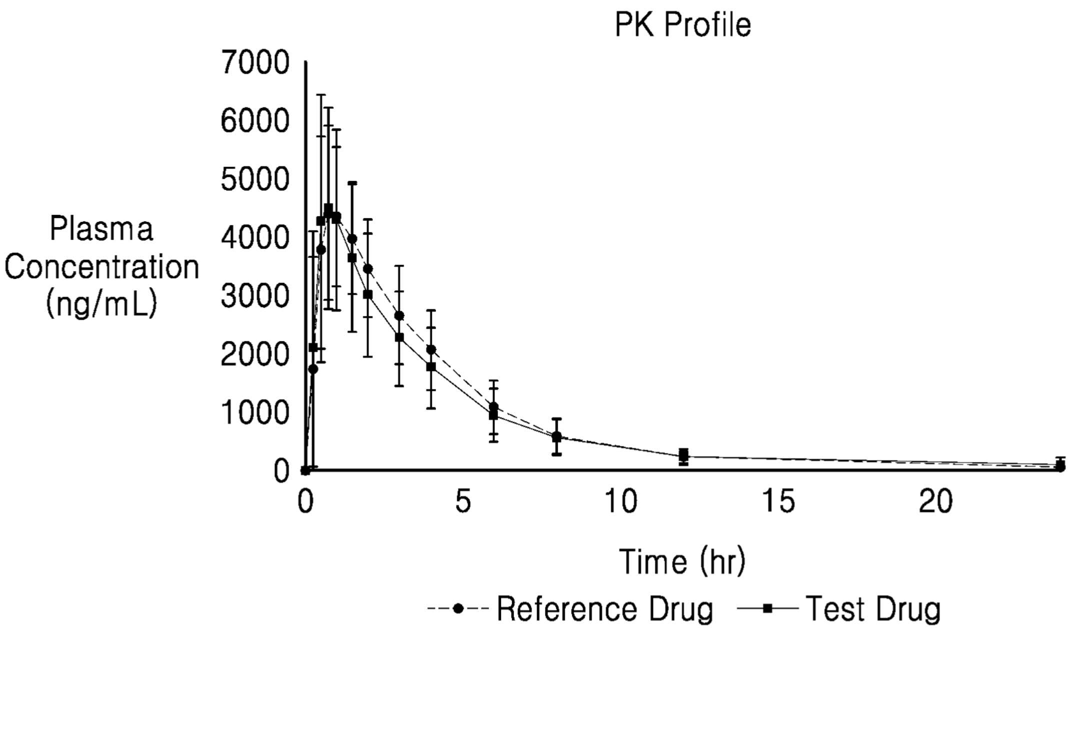
PK Profile
Plasma Concentration (ng/mL)
7000
6000
5000
4000
3000
2000
1000
0
0
5
10
15
20
Time (hr)
Reference Drug
Test Drug

**INTERNATIONAL SEARCH REPORT**

International application No. **PCT/KR2024/004667**

**A. CLASSIFICATION OF SUBJECT MATTER**

**C07D 405/12**(2006.01)i; **A61K 31/4184**(2006.01)i; **A61K 9/20**(2006.01)i; **A61K 9/00**(2006.01)i

According to International Patent Classification (IPC) or to both national classification and IPC

**B. FIELDS SEARCHED**

Minimum documentation searched (classification system followed by classification symbols)

C07D 405/12(2006.01); A61K 31/4184(2006.01); A61K 31/43(2006.01); C07D 235/06(2006.01); C07D 235/08(2006.01); C07D 311/22(2006.01); C07D 409/12(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 산 펌프 억제제(acid pump inhibitor), 4-[(5,7-다이플루오로-3,4-다이하이드로-2H-크로멘-4-일)옥시]-N,N,2-트라이메틸-1H-벤즈이미다졸-6-카복스아마이드(4-[(5,7-difluoro-3,4-dihydro-2H-chromen-4-yl)oxy]-N,N,2-trimethyl-1H-Benzimidazole-6-carboxamide), 염산염(hydrochloride)

**C. DOCUMENTS CONSIDERED TO BE RELEVANT**

| Category* | Citation of document, with indication, where appropriate, of the relevant passages | Relevant to claim No. |
|---|---|---|
| DX | KR 10-2008-0080195 A (PFIZER INC.) 02 September 2008 (2008-09-02)<br>See abstract; claims 1, 6, 8 and 9; example 1; and paragraphs [0001], [0075]-[0082], [0292], [0299], [0476] and [0477]. | 1-14 |
| A | KR 10-2020-0026118 A (HK INNO.N CORPORATION) 10 March 2020 (2020-03-10)<br>See entire document. | 1-14 |
| A | CN 112851646 A (INSTITUTE OF MATERIA MEDICA, CHINESE ACADEMY OF MEDICAL SCIENCES) 28 May 2021 (2021-05-28)<br>See entire document. | 1-14 |
| A | WO 2004-054984 A1 (ALTANA PHARMA AG et al.) 01 July 2004 (2004-07-01)<br>See entire document. | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 July 2024** | **15 July 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
**PCT/KR2024/004667**

**C. DOCUMENTS CONSIDERED TO BE RELEVANT**

| Category* | Citation of document, with indication, where appropriate, of the relevant passages | Relevant to claim No. |
|---|---|---|
| A | WO 2005-111000 A1 (ALTANA PHARMA AG et al.) 24 November 2005 (2005-11-24) See entire document. | 1-14 |

Form PCT/ISA/210 (second sheet) (July 2022)

**INTERNATIONAL SEARCH REPORT**
**Information on patent family members**

International application No.
**PCT/KR2024/004667**

| Patent document cited in search report | Publication date (day/month/year) | Patent family member(s) | Publication date (day/month/year) |
|---|---|---|---|
| KR 10-2008-0080195 A | 02 September 2008 | CA 2631880 A1 | 28 June 2007 |
| | | CA 2631880 C | 29 March 2011 |
| | | CN 101341149 A | 07 January 2009 |
| | | CN 101341149 B | 08 June 2011 |
| | | CN 102321077 A | 18 January 2012 |
| | | EP 1963311 A1 | 03 September 2008 |
| | | EP 1963311 B1 | 16 June 2010 |
| | | JP 2009-520017 A | 21 May 2009 |
| | | JP 2009-520017 A5 | 26 November 2009 |
| | | JP 4481344 B2 | 16 June 2010 |
| | | KR 10-1088247 B1 | 30 November 2011 |
| | | TW 200732326 A | 01 September 2007 |
| | | US 2007-0142448 A1 | 21 June 2007 |
| | | US 7723321 B2 | 25 May 2010 |
| | | WO 2007-072146 A1 | 28 June 2007 |
| KR 10-2020-0026118 A | 10 March 2020 | CN 112638378 A | 09 April 2021 |
| | | EP 3843723 A1 | 07 July 2021 |
| | | EP 3843723 A4 | 29 June 2022 |
| | | JP 2021-535910 A | 23 December 2021 |
| | | JP 7189327 B2 | 13 December 2022 |
| | | US 2021-0196685 A1 | 01 July 2021 |
| | | WO 2020-045992 A1 | 05 March 2020 |
| CN 112851646 A | 28 May 2021 | CN 112851646 B | 13 June 2023 |
| WO 2004-054984 A1 | 01 July 2004 | CN 100720236 A | 11 January 2006 |
| | | JP 2006-511600 A | 06 April 2006 |
| | | KR 10-2005-0084170 A | 26 August 2005 |
| | | TW 200504031 A | 01 February 2005 |
| | | US 2006-0194969 A1 | 31 August 2006 |
| WO 2005-111000 A1 | 24 November 2005 | AU 2005-243434 A1 | 24 November 2005 |
| | | CA 2566821 A1 | 24 November 2005 |
| | | EP 1756067 A1 | 28 February 2007 |
| | | JP 2007-538047 A | 27 December 2007 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007072146 A
- WO 2016117814 A
- WO 2018056697 A
- KR 1829706


### Non-patent literature cited in the description


- Remington's Pharmaceutical Science. Mack Publishing Company